# EUROPEAN PATENT SPECIFICATION

(11) **EP 3 102 600 B1**
(45) Date of publication and mention of the grant of the patent: **16.01.2019**
(21) Application number: 15704976.8
(22) Date of filing: 04.02.2015
(51) Int. Cl.: C07K 16/18, G01N 33/569

(54) **COMPOSITION AND METHOD FOR DETECTING MALIGNANT NEOPLASTIC DISEASE**
ZUSAMMENSETZUNG UND VERFAHREN ZUM NACHWEIS BÖSARTIGER TUMORERKRANKUNG
COMPOSITION ET PROCÉDÉ POUR DÉTECTER UNE MALADIE NÉOPLASTIQUE MALIGNE

(30) Priority: 05.02.2014 SE 1400058
(43) Date of publication of application: 14.12.2016
(73) Proprietor: Fujirebio Diagnostics Ab, 402 42 Göteborg (SE)
(72) Inventor: ÖHRVIK, Anders, 417 02 Göteborg (SE); LEVIN, Olle, 426 58 Västra Frölunda (SE)
(74) Representative: Valea AB
(86) International application number: PCT/EP2015/052325
(87) International publication number: WO 2015/118023

(56) References cited:
- WO-A1-2009/103542
- WO-A1-2014/004931
- WO-A2-2007/072220
- A DURNEZ ET AL: "The clinicopathological and prognostic relevance of cytokeratin 7 and 19 expression in hepatocellular carcinoma. A possible progenitor cell origin", HISTOPATHOLOGY, vol. 49, no. 2, 1 August 2006 (2006-08-01) , pages 138-151, XP055184687, ISSN: 0309-0167, DOI: 10.1111/j.1365-2559.2006.02468.x
- MAGALI FERRERO ET AL: "Flow Cytometric Analysis of DNA Content and Keratins by Using CK7, CK8, CK18, CKl9, and KLl Monoclonal Antibodies in Benign and Malignant Human Breast Tumors", CYTOMETRY, vol. 11, no. 6, 1 January 1990 (1990-01-01), pages 716-724, XP055185081,
- HABETS J M ET AL: "Absence of cytokeratin 8 and inconsistent expression of cytokeratins 7 and 19 in human basal cell carcinoma", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 8, no. 4, 1 July 1988 (1988-07-01), pages 611-616, XP009183896, ISSN: 0250-7005
- AHMAD WASEEM ET AL: "A Keratin Antibody Recognizing a Heterotypic Complex: Epitope Mapping to Complementary Locations on Both Components of the Complex", EXPERIMENTAL CELL RESEARCH, vol. 223, no. 2, 1 March 1996 (1996-03-01) , pages 203-214, XP055185562, ISSN: 0014-4827, DOI: 10.1006/excr.1996.0074
- Anonymous: "Fujirebio Diagnostics, Inc. : Rest of World Home : Products and Services : Manual Kits : Eia Kits (RUO)", , 26 January 2013 (2013-01-26), XP055185578, Retrieved from the Internet: URL:https://web.archive.org/web/2013012613 0614/http://www.fdi.com/world_home/product s/manual_kits/eia_kits/cyfra_21_1.html [retrieved on 2015-04-23]

## Description

### TECHNICAL FIELD

The present invention relates to the field of malignant neoplastic disease. Particularly, it relates to a composition and a method for an improved detection of malignant neoplastic disease, as well as its diagnostic and/or prognostic uses.

### BACKGROUND OF THE INVENTION

There is an immense need for simple, accurate and cost effective methods that can help diagnose malignant disease, to aid in treatment decisions and management of patients. Currently available tumor markers, with a few exceptions, have been reduced to therapy monitoring due to lack of sensitivity and specificity. Essentially all tumor markers are unsatisfactory as single markers and there is a need to complement established markers with new and more specific markers. Especially the lack of tumor specificity i.e.
increased marker values in various benign conditions such as liver chirrosis, kidney failure and general inflammation in various tissues is a fundamental problem of currently available tumor markers.

Keratins or fragments thereof circulating in serum, which are released from apoptotic or necrotic tumor and non-tumor cells, have been used as tumor markers for monitoring disease progression in several cancers. The expression of multiple keratins in an epithelial cell-specific manner and the fact that keratin expression profiles remain relatively stable during neoplastic transformation explains why keratins are commonly used tumor markers.

The most commonly used keratin tumor markers are tissue polypeptide antigen (TPA; a mixture of Keratin 8 (K8), Keratin 18 (K18), and Keratin 19 (K19)), tissue polypeptide-specific antigen (TPS; derived from K18), cytokeratin fragment 21-1 (CYFRA 21-1; derived from K19). TPA has been used as a potential serum marker to identify individuals with various epithelial cell-associated carcinomas, including those involving breast, colorectum, lung, and bladder. TPS has been used clinically in the care of individuals with cancers of the breast, ovary, prostate and CYFRA 21-1 in the care of individuals with lung cancers. The main potential clinical uses of TPA, TPS, and CYFRA 21-1 are to monitor treatment responses and tumor recurrence and to provide prognostic information. For the recommendation of routine use as a tumor marker, these markers have limited clinical utility because of their lack of organ specificity and elevated serum levels in nonmalignant diseases. High TPS and TPA levels are found in the context of several liver disorders and various inflammatory diseases. CYFRA 21-1 is the most specific of keratin markers and has gained widespread acceptance. However, high CYFRA levels accompany interstitial pulmonary fibrosis and renal failure. Also in cardiac heart failure CYFRA levels are leading to false positive results.

As mentioned above currently used tumor markers, not only CYFRA 21-1, give problems with false positive results but also e.g. Carcino Embryonal Antigen (CEA), Cancer Antigen 125 (CA125), Cancer Antigen 19-9 (CA 19-9), Cancer Antigen 15-3 (CA15-3), Neuron-Specific Enolase (NSE), Squamous Cell Carcinoma (SCC) to mention some. There is thus an urgent need to find better diagnostic and prognostic markers, means and methods when diagnosing and prognosing malignant neoplastic diseases in a simple and reliable way, as well as means to perform an accurate and less biased method or assay for detecting malignant neoplastic diseases. Accordingly, it is an object of the present invention to provide means and methods to perform accurate and less biased diagnostic assays, in a simple and efficient way for routine testing when diagnosing or prognosing malignant neoplastic diseases.

### SUMMARY OF THE INVENTION

The present invention is defined in the claims.

This object may be achieved by providing a composition comprising at least two antibodies, antigen-binding fragment(s), or combinations thereof, wherein at least one first antibody, antigen-binding fragment, or combination thereof recognizes a sequence located within the 256-412 amino acid sequence, or more preferably within the 300-380 amino acid sequence in the keratin 7 moiety of the heterotypic complex of keratin 7 with keratin 19 and/or fragment(s) thereof, and at least one second antibody, antigen-binding fragment, or combination thereof recognizes a sequence located within the 244-400 amino acid sequence, or more preferably within the 311-375 amino acid sequence in the keratin 19 moiety of the heterotypic complex of keratin 7 with keratin 19 and/or fragment(s) thereof, characterized in that said first and second antibodies, antigen-binding fragment(s), or combination thereof are capable of binding specifically and simultaneously to heterotypic complex of keratin 7 with keratin 19 and/or fragment(s) thereof.

The first and/or second targeting agents may be any kind of molecule that will recognize and bind to the targeted peptides or fragment(s) thereof. The first targeting agent recognizes a keratin 7 peptide and/or fragment(s) thereof. Keratin 7 (K7) is a protein that in humans is encoded by the *KRT7* gene. Keratin 7 is a type II keratin and is specifically expressed in the simple epithelia lining the cavities of internal organs and in the gland ducts and blood vessels. K7 is a 51 kDa protein, and is 469 amino acids long.

The second targeting agent recognizes a keratin 19 peptide and/or fragment(s) thereof. Keratin 19 (K19) is a protein that in humans is encoded by the *KRT19* gene. K19 is a type I keratin specifically found in the periderm, the transiently superficial layer that envelops the developing epidermis. K19 is a 44 kDa protein, and is 400 amino acids long.

The first and/or second targeting agents are advantageously selected from the group consisting of ligands, inhibitors, peptidomimetic compounds, peptides, proteins, antibodies, antigen-binding fragment(s) of antibodies, and/or combinations thereof. Both of the first and second targeting agents are capable of binding specifically and simultaneously to a heterotypic complex of keratin 7 with keratin 19, and/or fragment(s) thereof.

As used herein the term "heterotypic complex of keratin 7 with keratin 19" is intended to mean a molecular entity originating from a parallel and in register dimer between full-length keratin 7 and full-length keratin 19, and fragments thereof. The term further includes overlapping dimers between keratin 7 and keratin 19 which form anti-parallel tetramers between keratin 7 and keratin 19, and fragments thereof. It further includes oligomers, protofilaments and filaments assembled from these tetramers and any fragments thereof.

In the heterotypic complex of keratin 7 with keratin 19 there must be a minimum of least 3, preferably at least 5 or at least 10, or at least 15, or at least 20, or at least 25 or at least 30, but more preferably more than 40 amino acids in an unbroken sequence, derived from the keratin 7 moiety of the heterotypic complex, and there must be a minimum of least 3, preferably at least 5 or at least 10, or at least 15, or at least 20, or at least 25 or at least 30, but more preferably more than 40 amino acids in an unbroken sequence, derived from the keratin 19 moiety of the heterotypic complex. Said amino acids must be unique to keratin 7 or keratin 19 respectively.

Advantageously said minimum of least 3, preferably at least 5 or at least 10, or at least 15, or at least 20, or at least 25 or at least 30, but more preferably more than 40 amino acids in an unbroken sequence are from the 256-412 amino acid sequence in keratin 7. More preferably the unbroken sequence is from the 300-380 amino acid sequence in keratin 7. Advantageously said minimum of least 3, preferably at least 5 or at least 10, or at least 15, or at least 20, or at least 25 or at least 30, but more preferably more than 40 amino acids in an unbroken sequence are from the 244-400 amino acid sequence in keratin 19. More preferably the unbroken sequence is from the 311-375 amino acid sequence in keratin 19. When the first and second targeting agents bind specifically and simultaneously to said heterotypic complex of keratin 7 with keratin 19, and/or fragment(s) thereof, they will both be bound to the heterotypic complex of keratin 7 with keratin 19, and/or fragment(s) thereof and form a triplex comprising said first and second targeting agents and the heterotypic complex of keratin 7 with keratin 19, and/or fragment(s) thereof. Said first and second targeting agents will form said triplex with the heterotypic complex of keratin 7 with keratin 19, and/or fragment(s) thereof during a time period that is sufficiently long for the triplex to be detected by any means or methods that are described herein, or else by means or methods known to the person skilled in the art.

In one advantageous embodiment at least one of the first or second targeting agents is an antibody, antigen-binding fragment(s), or a variant, fusion, derivative or combination thereof. Advantageously both the first and second targeting agents are antibodies, antigen-binding fragment(s), or variants, fusions, derivatives or a combination thereof that will recognize and bind to heterotypic complex of keratin 7 with keratin 19, and/or fragment(s) thereof, and form an antigen-antibody triplex. Said antigen-antibody triplex thus comprises the heterotypic complex of keratin 7 with keratin 19, and/or fragment(s) thereof, to which both the first and the second antibodies, antigen-binding fragment(s), or variants, fusions, derivatives or a combination thereof are bound. The first antibody, antigen fragment(s), variant, fusion, derivative or combination thereof is bound to the keratin 7-peptide moiety of the heterotypic complex of keratin 7 with keratin 19, and/or fragment(s) thereof, and the second antibody, antigen fragment(s) or variant, fusion, derivative or a combination thereof is bound to the keratin 19-peptide moiety of the heterotypic complex of keratin 7 with keratin 19, and/or fragment(s) thereof.

When the first and second targeting agents are antibodies, antigen fragment(s) or variants, fusions, derivatives or a combination thereof, they will advantageously bind to different antigenic sites of the heterotypic complex of keratin 7 with keratin 19, and/or fragment(s) thereof. Typically the first antibody, antigen fragment(s) variant, fusion, derivative or combination thereof, recognizes and binds specifically to an antigenic site located on the keratin 7 moiety of the heterotypic complex of keratin 7 with keratin 19, and/or fragment(s) thereof. The second antibody, antigen fragment(s) variant, fusion, derivative or combination thereof, recognizes and binds specifically to an antigenic site located on the keratin 19 moiety of the heterotypic complex of keratin 7 with keratin 19, and/or fragment(s) thereof.

Said first and second targeting agents may bind to, and form a triplex with the heterotypic complex of keratin 7 with keratin 19, and/or fragment(s) thereof in any order. This means that in one embodiment the first targeting agent will first bind to the keratin 7 moiety of the heterotypic complex of keratin 7 with keratin 19, and/or fragment(s) thereof, and form a duplex. Thereafter the second targeting agent will bind to the keratin 19 moiety of said duplex and form a triplex.

In another embodiment the second targeting agent will first bind to the keratin 19-moiety of the heterotypic complex of keratin 7 with keratin 19, and/or fragment(s) thereof and form a duplex. Thereafter the first targeting agent will bind to the keratin 7-moiety of said duplex to form a triplex. In a further embodiment, both the first and second targeting agents will bind the heterotypic complex of keratin 7 with keratin 19, and/or fragment(s) thereof substantially at the same time.

In one advantageous embodiment the first and/or second targeting agent are primary antibodies, and or fragment(s) thereof, and in a further advantageous embodiment said first and/or second targeting agents are monoclonal or recombinant antibodies, and or fragment(s) thereof.

When the first targeting agent is an antibody, it is advantageously selected from the group consisting of the antibodies KS 7.18, and RCK105 or antibody fragment(s) variants, fusions, derivatives or combination thereof, which recognize a keratin 7 peptide and/or fragment(s) thereof, and has the capacity to bind specifically to heterotypic complex of keratin 7 with keratin 19, and/or fragment(s) thereof. Advantageously the first targeting agent recognizes a sequence of at least 3, or at least 5, or at least 7, or 10 or more amino acids located on the keratin 7 moiety of the heterotypic complex of keratin 7 with keratin 19, and which are unique to the keratin 7 protein sequence. Advantageously said first targeting agent recognizes a sequence located within the 256-412 amino acid sequence in the keratin 7 moiety of the heterotypic complex of keratin 7 with keratin 19. More preferably said sequence is from the 300-380 amino acid sequence in the keratin 7 moiety.

Advantageously the antibody recognizing the keratin 7 peptide or fragments thereof is the Ks 7.18 monoclonal antibody produced by clone Ks 7.18 which is available from Progen Biotechnik, GmBH, Germany. Monoclonal antibody KS 7.18 binds specifically to amino acids 300-350 of the K7 moiety of the heterotypic complex of keratin 7 with keratin 19, or fragment(s) thereof.

Alternatively the antibody recognizing the keratin 7 peptide or fragments thereof is the RCK105 monoclonal antibody produced by clone RCK105 which is available from Acris Antibodies Gmbh.

When the second targeting agent is an antibody, it is advantageously selected from the group consisting of the antibodies A53-B/A2.26 aka, Ks19.1, BM-19.21, and Ks19.2, or antibody fragment(s) variants, fusions, derivatives or combinations thereof, which recognize a keratin 19 peptide and/or fragment(s) thereof, and has the capacity to bind specifically to heterotypic complex of keratin 7 with keratin 19, and/or fragment(s) thereof. Advantageously the second targeting agent recognizes a sequence of at least 3, or at least 5, or at least 7, or 10 or more amino acids located on the keratin 19 moiety of the heterotypic complex of keratin 7 with keratin 19, and which are unique to the keratin 19 protein sequence. Advantageously said sequence is located within the 244-400 amino acid sequence in keratin 19. More preferably the sequence is from the 311-375 amino acid sequence in keratin 19.

Advantageously the antibody recognizing the keratin 19 peptide or fragment(s) thereof is the BM-19.21 monoclonal antibody produced by clone BM-19.21 which is available from Roche Diagnostics. BM 19.21 binds specifically to amino acids 346-367 of the of the K19 moiety of the heterotypic complex of keratin 7 with keratin 19.

Alternatively the antibody recognizing the keratin 19 peptide or fragment(s) thereof is the Ks 19.2 monoclonal antibody which is available from Progen Biotechnik, GmBH. Ks 19.2 binds specifically to amino acids 352-368 of the of the K19 moiety of the heterotypic complex of keratin 7 with keratin 19.

Alternatively the antibody recognizing the keratin 19 peptide or fragment(s) thereof is the Ks 19.1 monoclonal antibody produced by clone Ks 19.1 aka A53-B/A2. Ks 19.1 binds specifically to amino acids 311-335 of the of the K19 moiety of the heterotypic complex of keratin 7 with keratin 19.

Advantageously the first targeting agent is the Ks 7.18 monoclonal antibody and the second targeting agent is the BM-19.21 monoclonal antibody.

Alternatively the first targeting agent is the Ks 7.18 monoclonal antibody and the second targeting agent is the Ks 19.2 monoclonal antibody.

Alternatively the first targeting agent is the RCK105 monoclonal antibody and the second targeting agent is the Ks 19.1 monoclonal antibody.

A further aspect of the invention relates to an *in vitro* method for detecting a heterotypic complex of keratin 7 with keratin 19, and/or fragment(s) thereof in a biological sample, said method comprising the steps of:
a) contacting said biological sample with a composition comprising at least two antibodies, antigen-binding fragment(s), or combinations thereof, wherein at least one first antibody, antigen-binding fragment, or combination thereof recognizes a sequence located within the 256-412 amino acid sequence, or more preferably within the 300-380 amino acid sequence in the keratin 7 moiety of the heterotypic complex of keratin 7 with keratin 19 and/or fragment(s) thereof, and at least one second antibody, antigen-binding fragment, or combination thereof recognizes a sequence located within the 244-400 amino acid sequence, or more preferably within the 311-375 amino acid sequence in the keratin 19 moiety of the heterotypic complex of keratin 7 with keratin 19 and/or fragment(s) thereof, said first and second antibodies, antigen-binding fragment(s), or combination thereof-are capable of binding specifically and simultaneously to heterotypic complex of keratin 7 with keratin 19 and/or fragment(s) thereof; or alternatively
b) contacting said biological sample with a first antibody, antigen-binding fragment, or combination thereof recognizes a sequence located within the 256-412 amino acid sequence, or more preferably within the 300-380 amino acid sequence in the keratin 7 moiety of the heterotypic complex of keratin 7 with keratin 19 and/or fragment(s) thereof; and
c) contacting said biological sample with a second antibody, antigen-binding fragment, or combination thereof recognizes a sequence located within the 244-400 amino acid sequence, or more preferably within the 311-375 amino acid sequence in the keratin 19 moiety of the heterotypic complex of keratin 7 with keratin 19 and/or fragment(s) thereof; and
d) detecting said heterotypic complex of keratin 7 with keratin 19, and/or fragment(s) thereof; wherein steps b) and c) may be performed in any order or, simultaneously.

In an advantageous embodiment of the method, the composition as disclosed herein is used to contact a biological sample under suitable conditions. Examples of suitable conditions under which said composition and biological sample are contacted are described elsewhere herein below. Thereafter the heterotypic complex of keratin 7 with keratin 19, and/or fragment(s) thereof is detected by any suitable method also as described herein.

Alternatively the first and second targeting agents are provided separately and not as a composition. However, the biological sample may be contacted with said first and second targeting agents in any order. Therefore, in one embodiment the biological sample will first contact the first targeting agent which will bind to the keratin 7 moiety of a heterotypic complex of keratin 7 with keratin 19, and/or fragment(s) thereof present in the biological sample, and form a duplex between the first targeting agent and the heterotypic complex. Thereafter said duplex between the first targeting agent and the heterotypic complex thus formed will be contacted with the second targeting agent which will bind to the keratin 19 moiety of said formed duplex and thereby form a triplex comprising the first and second targeting agents bound to the heterotypic complex.

In another embodiment the biological sample will first contact the second targeting agent which will first bind to the keratin 19 moiety of the heterotypic complex of keratin 7 with keratin 19, and/or fragment(s) thereof and form a duplex between the second targeting agent and the heterotypic complex. Thereafter said duplex comprising the second targeting agent and the heterotypic complex thus formed will be contacted with the first targeting agent which will bind to the keratin 7 moiety of said formed duplex and thereby form a triplex comprising the first and second targeting agent and the heterotypic complex.

In a still further embodiment, both the first and second targeting agents will bind the heterotypic complex of keratin 7 with keratin 19, and/or fragment(s) thereof substantially at the same time.

Thereafter the heterotypic complex of keratin 7 with keratin 19, and/or fragment(s) thereof is detected by any suitable method as described below.

The first and second targeting agents used in the *in vitro* method are advantageously as described hereinabove.

The biological sample may be any sample selected from the group consisting of solid tissue samples such as e.g. biopsy specimens, tissue cultures or cells derived therefrom, and the progeny thereof, clinical samples, cells in culture, cell supernatants, cell lysates, or body fluids. Heterotypic complex of keratin 7 with keratin 19, and/or fragment(s) thereof may or may not be expressed in the biological sample.

Advantageously the method is used for biological fluid samples such as e.g. blood serum, blood plasma, lymph, exudates, feces, gastric acid, gastric juice, lymph, mucus, pericardial fluid, peritoneal fluid, pleural fluid, pus, saliva, sputum, synovial fluid, tears, sweat, vaginal secretion, vomit and urine. In a particularly advantageous embodiment the method is used for blood, blood plasma and or blood serum samples.

In an advantageous embodiment the method comprises a further step of comparing an expression of heterotypic complex of keratin 7 with keratin 19, and/or fragment(s) thereof in the biological sample to a positive and/or negative control, wherein the positive control comprises heterotypic complex of keratin 7 with keratin 19, and/or fragment(s) thereof, and the negative control does not comprise heterotypic complex of keratin 7 with keratin 19, and/or fragment(s) thereof.

In an advantageous embodiment said positive control is a biological sample obtained from a subject who is suffering from malignant neoplastic disease, and said negative control is a biological sample obtained from healthy subjects who are not suffering from malignant disease.

In a further embodiment the method may comprise a step of scoring the amount of heterotypic complex of keratin 7 with keratin 19, and/or fragment(s) thereof. The scoring may be done by means of the detected targeting agent complexes according to a standard scoring system known in the art or described herein.

Advantageously the method is performed on an automated reading device or the detection is performed manually.

One further aspect of the invention is a composition according to claims 1-6, for use in the *in vitro* method of claims 7-15, for
i) diagnosing and/or prognosing malignant neoplastic disease in a subject, and/or
ii) predicting efficacy of treatment of malignant neoplastic disease in a subject, and/or
iii) assessing outcome of treatment of malignant neoplastic disease in a subject, and/or
iv) assessing recurrence of malignant neoplastic disease in a subject
wherein the subject is a mammal having, or is suspected of having, a malignant neoplastic disease.

The subject is a mammal of the group consisting of humans, nonhuman primates such as chimpanzees and other apes and monkey species, farm animals such as cattle, sheep, pigs, goats and horses, domestic mammals such as dogs and cats, laboratory animals including rodents such as mice, rats and guinea pigs. Advantageously the subject is a mammal, including humans and non-human mammals. In the most advantageous embodiment, the subject is a human

The malignant neoplastic disease may be one of the group consisting of bile duct cancer (extrahepatic), bladder cancer, breast cancer, carcinoma of unknown (primary), cervical cancer, colon cancer, endometrial cancer, esophageal cancer, gallbladder cancer, gastric (stomach) cancer, head and neck cancer, hepatocellular (liver) cancer, hypopharyngeal cancer, kidney cancer, laryngeal cancer, liver cancer (primary), lung cancer (non-small cell), lung cancer (small cell), mesothelioma, non-small cell lung cancer, ovarian cancer, ovarian epithelial cancer (surface epithelial-stromal tumor), ovarian germ cell tumor, ovarian low malignant potential tumor, pancreatic cancer, prostate cancer, rectal cancer, renal cell carcinoma (kidney cancer), salivary gland cancer, small cell lung cancer, small intestine cancer, stomach cancer, testicular cancer, thyroid cancer, transitional cell cancer of the renal pelvis and ureter, or uterine cancer (endometrial).

Advantageously the malignant neoplastic disease is selected from the group consisting of lung cancer, bladder cancer, esophagus cancer, hepatocellular cancer, pancreatic cancer, gastric cancer and ovarian cancer. The method is particularly advantageous when the malignant neoplastic disease is selected from the group consisting of lung cancer, bladder cancer, esophagus cancer, and ovarian cancer.

When diagnosing and/or prognosing malignant neoplastic disease in a subject, the method comprises the steps of
a) obtaining a biological sample from a given subject
b) contacting said biological sample with a composition as described herein; or alternatively
c) contacting said biological sample with a first targeting agent recognizing a cytokeratin 7 peptide and/or fragment(s) thereof; and
d) contacting said biological sample with a second targeting agent recognizing a cytokeratin 19 peptide and/or fragment(s) thereof: and
e) detecting said heterotypic complex of cytokeratin 7 with cytokeratin 19, and/or fragment(s) thereof;
   wherein steps c) and d) may be performed in any order or, simultaneously; and
f) comparing the amount of heterotypic complex of cytokeratin 7 with cytokeratin19, and/or fragment(s) thereof detected to a positive and/or negative control, thereby diagnosing and/or prognosing the malignant neoplastic disease in the subject.

Optionally, a scoring may be done of the detected antigen-antibody complexes according to a standard scoring system known in the art or described herein.

The sample may be any sample possibly comprising malignant neoplastic disease. Further embodiments are wherein the positive control comprises cells from a subject who is suffering from the malignant neoplastic disease. Even further embodiments are wherein the negative control comprises cells from healthy subjects who are not suffering from malignant neoplastic disease.

When predicting outcome of treatment in a subject suffering from malignant neoplastic disease, the method comprises the steps of
a) obtaining a biological sample from a subject suffering from malignant neoplastic disease
b) detecting the expression of heterotypic complex of keratin 7 with keratin 19 in said biological sample
c) comparing the expression of heterotypic complex of keratin 7 with keratin 19 to a positive and/or negative control, and thereby predicting the outcome of treatment of the malignant neoplastic disease in said subject based on the detected expression of heterotypic complex of keratin 7 with keratin 19, and/or fragments thereof.

When assessing efficacy of treatment of malignant neoplastic disease in a subject who is being treated for malignant neoplastic disease, the method comprises the steps of
a) obtaining a biological sample from a subject who is undergoing treatment for malignant neoplastic disease
b) detecting the expression of heterotypic complex of keratin 7 with keratin 19 in said biological sample
c) repeating steps a) and b) at one or more time points during treatment of said subject for malignant neoplastic disease, and wherein a change in relative expression of heterotypic complex of keratin 7 with keratin 19 over time indicates the efficacy of treatment.

Thus, an indication of effective treatment is a relative change in decreasing expression of heterotypic complex of keratin 7 with keratin 19 relative a previous sample analyzed in the steps of repeating the method.

Optionally, a scoring may be done of the detected heterotypic complex of keratin 7 with keratin 19 according to a standard scoring system known in the art or described herein. The sample may be any sample possibly comprising malignant neoplastic disease, preferably a biological sample from a subject having malignant neoplastic disease, and that subject will be, is in-between or is currently under treatment.

When assessing recurrence of malignant neoplastic disease, the method comprises the steps of
a) obtaining a biological sample from a subject having previously had malignant neoplastic disease,
b) detecting the expression of heterotypic complex of keratin 7 with keratin 19 in said biological sample,
c) repeating steps a) and b) at one or more time points post treatment of said subject for malignant neoplastic disease, and wherein a change in relative expression of heterotypic complex of keratin 7 with keratin 19 over time may indicate recurrence of malignant neoplastic disease.

Thus, an indication of recurrence is a relative change in increasing amounts of heterotypic complex of keratin 7 with keratin 19 that identify malignant neoplastic disease, i.e. an over-time increase in expression of protein marker heterotypic complex of keratin 7 with keratin 19 relative a previous sample analyzed in the steps of repeating the method.

Further disclosed is the use of the *in vitro* method to
i) detect heterotypic complex of keratin 7 with keratin 19 and or fragment(s) thereof, and/or
ii) detect malignant neoplastic disease; and/or
iii) diagnose or prognose malignant neoplastic disease in a subject, and/or
iv) predict outcome of treatment of malignant neoplastic disease in a subject, and/or
v) assess efficacy of treatment of malignant neoplastic disease in a subject, and/or
vi) assess recurrence of malignant neoplastic disease in a subject.

In a further aspect the invention provides a kit for
i) detecting heterotypic complex of keratin 7 with keratin 19, and/or fragment(s) thereof in a biological sample; and/or
ii) detecting malignant neoplastic disease in a subject; and/or
iii) diagnosing or prognosing malignant neoplastic disease in a subject; and/or
iv) predicting outcome of treatment of malignant neoplastic disease in a subject; and/or
v) assessing efficacy of treatment of malignant neoplastic disease in a subject; and/or
vi) assessing recurrence of malignant neoplastic disease in a subject;
the kit comprising:
a) a composition as defined in any one of claims 1-6; or alternatively
b) a first container comprising a first targeting agent recognizing a keratin 7 peptide and/or fragment(s) thereof; and
c) a second container comprising a second targeting agent recognizing a keratin 19 peptide and/or fragment thereof, and
d) optionally instructions for performing a method as defined in any one of claims 7-15.

### BRIEF DESCRIPTION OF THE FIGURES

Figure 1 shows keratin protein structure and filament assembly.
Figure 2 shows a typical calibration curve for the K7/19 assay.
Figure 3 shows the sensitivity vs 95% specificity for benign diseases as assayed by the method of the invention.
Figure 4 shows a ROC-curve comparing the different tumor markers, CA125, HE4, K7/19 and CYFRA in ovarian cancer.
Figure 5 shows ROC-curve comparing the combination CA125EIA & HE4 EIA with the combination CA125EIA & K7/19 assay.

### DETAILED DESCRIPTION OF THE INVENTION

Before the present invention is described, it is to be understood that this invention is not limited to the particular embodiments described as methods, devices, and formulations may of course vary. It is also to be understood that the terminology used herein is for the purpose of describing particular embodiments only, and is not intended to limit the scope of the present invention which will be limited only by the appended claims. It must be noted that as used herein and in the appended claims, the singular forms "a," "an," and "the" include plural referents unless the context clearly dictates otherwise, and includes reference to equivalent steps and methods known to those skilled in the art.

The terms used in this invention are, in general, expected to adhere to standard definitions generally accepted by those having ordinary skill in the art of molecular biology. A few exceptions, as listed below, have been further defined within the scope of the present invention.

"At least one" as used herein means one or more, i.e. 1, 2, 3, 4, 5, 6, 7, 8, 9, 10 etc.

"Detection", "detect", "detecting" as used herein includes qualitative and/or quantitative detection (measuring levels) with or without reference to a control, and further refers to the identification of the presence, absence, or quantity of a given protein, specifically heterotypic complex of keratin 7 with keratin 19, and/or fragment(s) thereof.

"Diagnosis" as used herein encompasses the identification of the nature of a disease.

"Prognosis" as used herein encompasses a forecast as to the probable outcome of a disease, the prospects as to recovery from a disease as indicated by the nature and symptoms of a disease.

"True positives" refers to those subjects having a localized or a metastasized malignant neoplasm.

"False negatives" refers to those subjects having either a localized or a metastasized malignant neoplasm and are not categorized as such by a diagnostic assay.

"True negatives" refers to those subjects who do not have a localized or a metastasized malignant neoplasm and who are categorized as such by a diagnostic assay.

"False positives" refers to those subjects who do not have a localized or a metastasized malignant neoplasm but are categorized by a conventional diagnostic assay as having a localized or metastasized malignant neoplasm.

Depending on context, the term "false positives" may also refer to those subjects who do not have malignant neoplasm but are categorized by a diagnostic assay as having malignant neoplasm or a non-malignant disease.

"Sensitivity", as used herein in the context of its application to diagnostic assays, refers to the proportion of all subjects with localized or metastasized malignant neoplasm that are correctly identified as such (that is, the number of true positives divided by the sum of the number of true positives and false negatives).

"Specificity" of a diagnostic assay, as used herein in the context of its application to diagnostic assays, refers to the proportion of all subjects with neither localized or metastasized malignant neoplasm that are correctly identified as such (that is, the number of true negatives divided by the sum of the number of true negatives and false positives).

The terms "neoplasm" or "tumor" may be used interchangeably and refer to an abnormal mass of tissue wherein the growth of the mass surpasses and is not coordinated with the growth of normal tissue. A neoplasm or tumor may be defined as "benign" or "malignant" depending on the following characteristics: degree of cellular differentiation including morphology and functionality, rate of growth, local invasion and metastasis. A "benign" neoplasm is generally well differentiated, has characteristically slower growth than a malignant neoplasm and remains localized to the site of origin. In addition a benign neoplasm does not have the capacity to infiltrate, invade or metastasize to distant sites.

A "malignant" neoplasm is generally poorly differentiated (anaplasia), has characteristically rapid growth accompanied by progressive infiltration, invasion, and destruction of the surrounding tissue. Furthermore, a malignant neoplasm has the capacity to metastasize to distant sites. The term "metastasis" refers to the spread or migration of cancerous cells from a primary (original) tumor to another organ or tissue, and is typically identifiable by the presence of a "secondary tumor" or "secondary cell mass" of the tissue type of the primary (original) tumor and not of that of the organ or tissue in which the secondary (metastatic) tumor is located. For example a carcinoma of the lung that has migrated to bone is said to be metastasized lung cancer, and consists of cancer cells originating from epithelial lung cells growing in bone tissue.

"Healthy" refers to a subject possessing good health. Such a subject demonstrates an absence of any malignant or non-malignant disease. In the context of this application, a "healthy individual" is only healthy in that they have an absence of any malignant or non-malignant disease; a "healthy individual" may have other diseases or conditions that would normally not be considered "healthy".

"Subject" as used herein includes humans, nonhuman primates such as chimpanzees and other apes and monkey species, farm animals such as cattle, sheep, pigs, goats and horses, domestic mammals such as dogs and cats, laboratory animals including rodents such as mice, rats and guinea pigs, and the like. The term does not denote a particular age or sex. Thus, adult and newborn subjects, as well as fetuses, whether male or female, are intended to be covered. In preferred embodiments, the subject is a mammal, including humans and non-human mammals. In the most preferred embodiment, the subject is a human.

"Monoclonal antibody" or "mAb" as used herein refers to an antibody of a single amino acid composition, that is directed against a specific antigen and that is produced by a single clone of B cells or hybridoma.

"Polyclonal antibody" as used herein refers to an antibody that is directed against a specific antigen that is derived from different B-cell lines.

"Fab" as used herein refers to an antibody fragment having a molecular weight of about 50,000 Da and antigen binding activity, in which about a half of the N-terminal side of the H chain and the entire L chain, among fragments obtained by treating IgG with a protease, papaine, are bound together through a disulfide bond.

"F(ab')2" as used herein refers to an antibody fragment having a molecular weight of about 100,000 Da and antigen binding activity, which is slightly larger than the Fab bound via a disulfide bond of the hinge region, among fragments obtained by treating IgG with a protease, pepsin.

"Fab¹" as used herein refers to an antibody fragment having a molecular weight of about 50,000 Da and antigen binding activity, which is obtained by cutting a disulfide bond of the hinge region of the F(ab')2. As used herein, a single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, preferably by using gene recombination techniques.

"Hybridoma" as used herein denotes a cell, which is obtained by subjecting a B cell prepared by immunizing a non-human mammal with an antigen to cell fusion with a myeloma cell derived from a mouse or the like which produces a desired monoclonal antibody having an antigen specificity.

The terms "polypeptide," "protein," and "peptide" are used herein interchangeably to refer to amino acid chains in which the amino acid residues are linked by peptide bonds or modified peptide bonds. The amino acid chains can be of any length of greater than two amino acids. Unless otherwise specified, the terms "polypeptide," "protein," and "peptide" also encompass various modified forms thereof. Such modified forms may be naturally occurring modified forms or chemically modified forms. Examples of modified forms include, but are not limited to, glycosylated forms, phosphorylated forms, myristoylated forms, palmitoylated forms, ribosylated forms, acetylated forms, ubiquitinated forms, etc. Modifications also include intra-molecular crosslinking and covalent attachment to various moieties such as lipids, flavin, biotin, polyethylene glycol or derivatives thereof, etc. In addition, modifications may also include cyclization, branching and cross-linking. Further, amino acids other than the conventional twenty amino acids encoded by genes may also be included in a polypeptide

As used herein a "biological sample" encompasses a variety of sample types obtained from any subject having or not having malignant neoplasm. A typical subject is a human; however, any mammal that has a malignant neoplasm that may develop cancer can serve as a source of a biological sample useful in a disclosed method. Exemplary biological samples useful in the disclosed methods include but are not limited to solid tissue samples such as e.g. biopsy specimens, tissue cultures or cells derived there from, and the progeny thereof, clinical samples, cells in culture, cell supernatants, cell lysates, body fluids. For example, biological samples include samples obtained from a tissue or fluids collected from an individual suspected of having a malignant neoplasm.

Examples of biological fluid samples include but are not limited to blood serum, blood plasma, lymph, exudates, feces, gastric acid, gastric juice, lymph, mucus, pericardial fluid, peritoneal fluid, pleural fluid, pus, saliva, sputum, synovial fluid, tears, sweat, vaginal secretion, vomit and urine.

Further examples are biopsies and/or fine needle aspirates. Samples may be fresh or processed post-collection (e.g., for archiving purposes). In some examples, processed samples may be fixed (e.g., formalin-fixed) and/or wax- (e.g., paraffin-) embedded. Fixatives for mounted cell and tissue preparations are well known in the art and include, without limitation, 95% alcoholic Bouin's fixative; 95% alcohol fixative; B5 fixative, Bouin's fixative, formalin fixative, Karnovsky's fixative (glutaraldehyde), Hartman's fixative, Hollande's fixative, Orth's solution (dichromate fixative), and Zenker's fixative (see, e.g., Carson, Histotechology: A Self-Instructional Text, Chicago: ASCP Press, 1997). In some examples, the sample (or a fraction thereof) is present on a solid support.

Solid supports useful in a disclosed method need only bear the biological sample and, optionally, but advantageously, permit convenient detection of the proteins of interest in the sample. Exemplary supports include microscope slides (e.g., glass microscope slides or plastic microscope slides), coverslips (e.g., glass coverslips or plastic coverslips), tissue culture dishes, multi-well plates, membranes (e.g., nitrocellulose or polyvinylidene fluoride (PVDF)) or BIACORE®; chips.

"Treatment" as used herein is defined as the management of a patient through medical or surgical means. The treatment improves or alleviates at least one symptom of a medical condition or disease and is required to provide a cure. The term "treatment outcome" or "outcome of treatment" as used herein is the physical effect upon the patient of the treatment.

The term "algorithm" as used herein refers to a mathematical formula that provides a relationship between two or more quantities. Such a formula may be linear, or non-linear, and may exist as various numerical weighting factors in computer memory.

"Lung cancer" refers to a neoplasm, e.g., malignant neoplasm, of the lung within a given subject, wherein the neoplasm is of epithelial origin i.e. carcinoma of the lung. Lung carcinomas are categorized by the size and appearance of the malignant cells and the term "lung cancer" includes both non-small cell lung cancer (NSCLC) and small cell lung cancer (SCLC). The term "lung cancer", when used without qualification, includes both localized and metastasized lung cancer. The term "lung cancer" can be qualified by the terms "localized" or "metastasized" to differentiate between different types of tumor, where "localized" refers to the original mother tumor, and the metastasized to the tumors that has spread from the original mother tumor.

The TNM System (tumor, node, metastases) may be used to stage NSCLC in an initial evaluation. Using the TNM descriptors, a group is assigned, ranging from occult cancer, through stages 0, IA (one-A), IB, IIA, IIB, IIIA, IIIB and IV (four). This stage group assists with the choice of treatment and estimation of prognosis

Small-cell lung carcinoma has traditionally been classified as 'limited stage' (confined to one half of the chest and within the scope of a single tolerable radiotherapy field) or "extensive stage" (more widespread disease). However, the TNM classification and grouping are useful in estimating prognosis.

For both NSCLC and SCLC, the two general types of staging evaluations are clinical staging and surgical staging. Clinical staging is performed prior to definitive surgery. It is based on the results of imaging studies (such as CT scans and PET scans) and biopsy results. Surgical staging is evaluated either during or after the operation, and is based on the combined results of surgical and clinical findings, including surgical sampling of thoracic lymph nodes

"Ovarian cancer" refers to a neoplasm, e.g., malignant neoplasm, of the ovary within a given female subject, wherein the neoplasm is of epithelial origin.

The term "ovarian cancer", when used without qualification, includes both localized and metastasized ovarian cancer. The term "ovarian cancer" can be qualified by the terms "localized" or "metastasized" to differentiate between different types of tumor, where "localized" refers to the original mother tumor, and the metastasized to the tumors that has spread from the original mother tumor.

Ovarian cancer can be staged according to the AJCC/TNM System. This describes the extent of the primary tumor (T), the absence or presence of metastasis to nearby lymph nodes (N), and the absence or presence of distant metastasis (M). The extent of primary tumor contains three sub-categories T1, T2, T3. This closely resembles the system that is actually used by most gynecologic oncologists, called the FIGO system. Both rely on the results of surgery for the actual stage. In the FIGO system the tumor stage is classified from I-IV depending on how far the tumor has spread. Where stage IV is worst meaning that the tumor has spread to its estimated limit. Stages I-III contains the sub-categories A, B and C.

"Esophagus cancer" refers to a neoplasm, e.g., malignant neoplasm, of the esophagus within a given subject, wherein the neoplasm is of epithelial origin i.e. carcinoma of the esophagus. These carcinomas fall into two classes: either adenocarcinomas or squamous cell carcinomas.

The most common system used to stage esophageal cancer is the TNM system of the American Joint Committee on Cancer (AJCC). The TNM system is based on several key pieces of information: T refers to how far the primary tumor has grown into the wall of the esophagus and into nearby organs. N refers to cancer spread to nearby lymph nodes. M indicates whether the cancer has metastasized (spread to distant organs). G describes the grade of the cancer, which is based on how the patterns of cancer cells look under a microscope. Staging also takes into account the cell type of the cancer (squamous cell carcinoma or adenocarcinoma). For squamous cell cancers, the location of the tumor can also be a factor in staging.

"Bladder cancer" refers to a neoplasm, e.g., malignant neoplasm, of the urinary bladder within a given subject, wherein the neoplasm originates from the urothelium or transitional epithelium. Bladder cancer refers thus to Transitional cell (urothelial) carcinoma, but it also refers to adenocarcinoma of the bladder.

The staging system most often used for bladder cancer is that of the American Joint Committee on Cancer (AJCC). This is also called the TNM system. The T category describing the tumor contains the sub-categories: T0, Ta, Tis, T1, T2a, T2b, T3a, T3b, T4.

### Immunoassays such as immunohistochemistry (IHC) and immunocytochemistry (ICC)

As used herein an "immunoassay" is a biochemical test that measures the presence or concentration of a substance in samples that frequently contain a complex mixture of substances. Such assays are based on the unique ability of an antibody to bind with high specificity to one or a very limited group of molecules or antigens. Immunohistochemistry (IHC) and immunocytochemistry (ICC) are two exemplary immunoassay techniques useful for detecting the antigen heterotypic complex of keratin 7 with keratin 19, and/or fragment(s) thereof in the disclosed methods and uses.

Immunocytochemistry (ICC) is a common laboratory technique that uses antibodies that target specific peptides or protein antigens in the cell via specific epitopes. These bound antibodies can then be detected using several different methods. ICC allows researchers to evaluate whether or not cells in a particular sample express the antigen in question. In cases where an immunopositive signal is found, ICC also allows researchers to determine which sub-cellular compartments are expressing the antigen.

In immunohistochemistry (IHC), samples are sections of biological tissue, where each cell is surrounded by tissue architecture and other cells normally found in the intact tissue. Immunocytochemistry is a technique used to assess the presence of a specific protein or antigen in cells (cultured cells, cell suspensions) by use of a specific antibody, which binds to it, thereby allowing visualization and examination under a microscope. It is a valuable tool for the determination of cellular contents from individual cells. Samples that can be analyzed include blood smears, aspirates, swabs, cultured cells, and cell suspensions.

Enzyme-linked immunosorbent assay (ELISA) and sandwich ELISA, are immunoassays that are advantageously used in the methods disclosed herein. In a (direct) ELISA an unknown amount of antigen is affixed to a surface, and then a specific antibody is applied over the surface so that it can bind to the antigen. This antibody is linked to an enzyme, and in the final step a substance is added so that the enzyme can convert to some detectable signal, most commonly a colour change in a chemical substrate. In a sandwich ELISA a capture antibody that can bind to the antigen is affixed to the surface. The other steps are equivalent to the ELISA.

In an Enzyme Immuno Assay (EIA), which is similar to the sandwich ELISA, streptavidin is affixed to a surface and then the capture antibody is biotinylated, otherwise the other steps are performed equivalently as the ELISA. The EIA immunoassay is advantageously used in the methods disclosed herein.

Western Blot (sometimes called the protein immunoblot) is a widely used analytical technique used to detect specific proteins in the given sample of tissue homogenate or extract. It uses gel electrophoresis to separate native or denatured proteins by the length of the polypeptide (denaturing conditions) or by the 3-D structure of the protein (native/ non-denaturing conditions). The proteins are then transferred to a membrane (typically nitrocellulose or PVDF), where they are probed (detected) using antibodies specific to the target protein.

Targeting agents such as e.g. antibodies, antigen-binding fragments, or variants, fusions or derivatives specific for keratin 7 and keratin 19 and/or fragments thereof are used to detect the expression of heterotypic complex of keratin 7 with keratin 19, and/or fragment(s) thereof. The targeting agents of the invention are advantageously provided as a composition but may also be provided separately but subsequent to one another. The *in vitro* method of the invention thus provides antibodies antigen-binding fragments, or variants, fusions or derivatives binding to heterotypic complex of keratin 7 with keratin 19, and/or fragment(s) thereof. The antibodies can be detected, as further described herein, by direct labelling of the antibodies themselves, for example, with radioactive labels, fluorescent labels, hapten labels such as, biotin, or an enzyme such as horseradish peroxidase or alkaline phosphatase. Alternatively, an indirect labelling is used where the unlabeled primary antibody is used in conjunction with a labelled secondary antibody, comprising e.g. antiserum, polyclonal antiserum or a monoclonal antibody specific for the primary antibody. IHC and ICC protocols are well known in the art and are commercially available, see e.g. Antibodies: A Laboratory Manual, Harlow and Lane (Cold Spring Harbor Laboratory press, Cold Spring Harbor, NY 1988) and Current Protocols in Immunology, and Current Protocols in Molecular Biology, both John Wiley and Sons, Inc., N. Y.).

As revealed above, the present invention provides means and methods to improve sensitivity and specificity of the diagnosis and/or prognosis of malignant neoplastic disease such as e.g. lung cancer, bladder cancer, esophagus cancer and/or ovarian cancer. More specifically, the present invention provides a composition that gives better sensitivity for detection of malignant neoplastic disease so as to improve the detection of malignant neoplastic protein markers in ELISA and EIA as well as in IHC and ICC, thereby giving a more consistent and reliable result when performing diagnosis and/or prognosis of patients having a malignant neoplastic disease such as e.g. lung cancer, bladder cancer, esophagus cancer and/or ovarian cancer.

Further, the methods according to the invention will improve the identification of malignant neoplastic diseases such as e.g. lung cancer, bladder cancer, esophagus cancer and/or ovarian cancer compared to available methods.

The targeting agents used in the methods herein thus show an improved detection of various malignant neoplastic diseases when compared to other known methods such as e.g. CYFRA 21-1 (see Examples given herein).

Examples 2-5 show that malignant neoplastic disease such as e.g. lung cancer, bladder cancer, esophagus cancer and/or ovarian cancer are identified by an expression of heterotypic complex of keratin 7 with keratin 19, and/or fragment(s) thereof.

The method detects malignant neoplastic disease such as e.g. lung cancer, bladder cancer, esophagus cancer and/or ovarian cancer with antibodies binding specifically to the heterotypic complex of keratin 7 with keratin 19, and/or fragment(s) thereof.

### The proteins

The method of the present invention encompass the use of at least two targeting agents such as e.g. two primary antibodies, antigen-binding fragments, variants, fusions, derivatives or fragments thereof. At least one first primary antibody binds specifically to keratin 7 or fragments thereof and at least one second primary antibody binds specifically to keratin 19 or fragments thereof. Both of said first and second primary antibodies are capable of binding specifically and simultaneously to Heterotypic complex of keratin 7 with keratin 19, and/or fragment(s) thereof and/or fragments thereof.

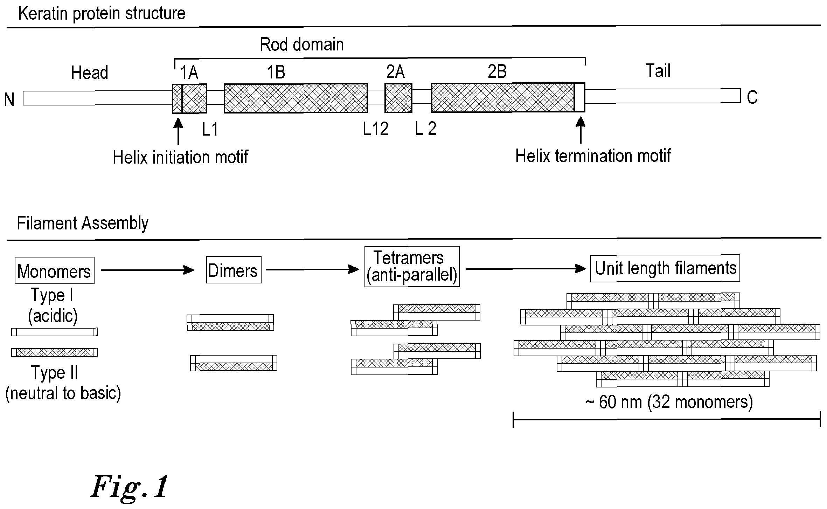

Keratin 7 (K7) (NCBI Reference Sequence: NP_005547.3, NCBI gene ID: 3855) also known as cytokeratin 7 (CK7) or sarcolectin (SCL) is a protein that in humans is encoded by the *KRT7* gene. Keratin 7 is a type II keratin and is together with other genes encoding the type II keratins clustered in a region of chromosome 12q12-q13. K7 is a 51 kDa protein which is 469 amino acids long and has the general structure as seen in Fig. 1. Alternative splicing may result in several transcript variants. It is specifically expressed in the simple epithelia lining the cavities of the internal organs and in the gland ducts and blood vessels).

Keratin 19 (K19) (NCBI Reference Sequence: NP_002267.2, NCBI gene ID: 3880) is also known as cytokeratin 19 (CK19) and is a type I keratin specifically found in the periderm, the transiently superficial layer that envelops the developing epidermis. K19 is a 44 kDa protein which is 400 amino acids long and has the general structure as seen in Fig. 1. K19 is in humans encoded by the *KRT19* gene and is located in a region of chromosome 17q12-q21 gene.

In vivo keratins assemble as heteropolymers, i.e. a Type I and a Type II protein form heterodimers, which in turn assemble to form tetramers. Two monomers form a parallel dimer by the winding of their α-helical rods into a coiled coil, oriented in register and in the same direction, and then two dimers join side-by-side in a staggered anti-parallel orientation to form a bidirectional tetramer (see Fig. 1). When keratins K7 and K19 assemble into heterodimers and tetramers heterotypic complexes of keratin 7 with keratin 19 are formed.

As used herein the term "heterotypic complex of keratin 7 with keratin 19" is intended to include a molecular entity originating from a parallel and in register dimer between full-length keratin 7 and full-length keratin 19 and fragments thereof. It further includes overlapping dimers between keratin 7 and keratin 19 into anti-parallel tetramers between keratin 7 and keratin 19 and fragments thereof. It further includes oligomers, protofilaments and filament assembled from these tetramers and any fragments thereof. For a general structure of heterotypic complex of keratins see Fig. 1.

In the heterotypic complex of keratin 7 with keratin 19 there is advantageously a minimum of least 3, preferably at least 5 or at least 10, or at least 15, or at least 20, or at least 25 or at least 30, but more preferably more than 40 amino acids in an unbroken sequence, derived from the keratin 7 moiety of the heterotypic complex, and there must be a minimum of least 3, preferably at least 5 or at least 10, or at least 15, or at least 20, or at least 25 or at least 30, but more preferably more than 40 amino acids in an unbroken sequence, derived from the keratin 19 moiety of the heterotypic complex. Said amino acids must be unique to keratin 7 or keratin 19 respectively.

Advantageously said minimum of least 3, preferably at least 5 or at least 10, or at least 15, or at least 20, or at least 25 or at least 30, but more preferably more than 40 amino acids in an unbroken sequence are from the 256-412 amino acid sequence in the keratin 7 protein. More preferably the unbroken sequence is from the 300-380 amino acid sequence in keratin 7.

Advantageously said minimum of least 3, preferably at least 5 or at least 10, or at least 15, or at least 20, or at least 25 or at least 30, but more preferably more than 40 amino acids in an unbroken sequence are from the 244-400 amino acid sequence in the keratin 19 protein. More preferably the unbroken sequence is from the 311-375 amino acid sequence in keratin 19. The antibodies Herein disclosed is a method encompassing the use of at least two targeting agents, such as e.g. two primary antibodies, antigen-binding fragments, or variants, fusions or derivatives thereof to detect the presence of heterotypic complex of keratin 7 with keratin 19, and/or fragment(s) thereof in a biological sample, wherein a first primary antibody, antigen-binding fragment, or variant, fusion or derivative thereof binds specifically to the keratin 7 or fragments thereof of the heterotypic complex, and a second primary antibody, antigen-binding fragment, or variant, fusion or derivative thereof binds specifically to the keratin 19 or fragments thereof of the heterotypic complex. Advantageously, the at least two antibodies, antigen-binding fragments, or variants, fusions or derivatives thereof are present together in an antibody cocktail, either in a format ready-to-use by the user or in a concentrated solution which requires a dilution before its use, sometimes referred to as a stock solution.

Thus, one aspect of the disclosure encompasses a composition comprising at least one first primary antibody, antigen-binding fragment, or variant, fusion or derivative thereof that recognizes a keratin 7 peptide and/or fragment(s) thereof, and at least one second primary antibody, antigen-binding fragments or variants fusions or derivative thereof that recognizes a keratin 19 peptide and/or fragment(s) thereof, wherein both the first and the second primary antibodies are capable of binding specifically and simultaneously to heterotypic complex of keratin 7 with keratin 19, and/or fragment(s) thereof.

By "binding specifically to" or "reacting specifically with" as used herein it is intended to equal "capable of binding selectively" or "binding specifically to". As used herein the expressions are intended to mean that the antibody or antigen-binding fragment, or variant, fusion or derivative thereof, including any anti-body derived binding moiety, which is capable of binding to an antigen of a molecule and further which binds at least 10-fold more strongly the proteins than to another proteins for example at least 50-fold more strongly or at least 100-fold more strongly. The binding moiety may be capable of binding selectively to the protein under physiological conditions, e.g. in vivo. Suitable methods for measuring relative binding strengths include, immunoassays, for example where the binding moiety is an antibody. Alternatively, binding may be assessed using competitive assays or using Biacore(R) analysis (Biacore International AB, Sweden).

By the term "binding specifically and simultaneously to" as used herein it is intended to mean that the first and second targeting agents bind specifically and simultaneously to the heterotypic complex of keratin 7 with keratin 19, and/or fragment(s) thereof, and form a triplex comprising said first and second targeting agents and the heterotypic complex of keratin 7 with keratin 19, and/or fragment(s) thereof. Said first and second targeting agents will form said triplex with the heterotypic complex of keratin 7 with keratin 19, and/or fragment(s) thereof during a time period that is sufficiently long for the complex to be detected by any means or methods that are described herein, or else by means or methods known to the person skilled in the art.

In one aspect embodiment, each of the first and second antibodies or antigen-binding fragments, or variants, fusions or derivatives thereof, bind to separate antigenic sites of the heterotypic complex of keratin 7 with keratin 19, and/or fragment(s) thereof. Typically the first primary antibody reacts specifically with an antigenic site present on the K7 moiety of the heterotypic complex of keratin 7 with keratin 19, and/or fragment(s) thereof, and the second primary antibody reacts specifically with an antigenic site present on the K19 moiety of the heterotypic complex of keratin 7 with keratin 19, and/or fragment(s) thereof.

Because the two monomers keratin 7 and keratin 19 of the heterotypic complex between keratin 7 and keratin 19 are aligned in parallel and in register with the helical structures, any two antibodies binding to corresponding positions starting from helix 1A to helix 2B (see Fig. 1), on keratin 7 and keratin 19 respectively will not bind simultaneously. This means that where the keratin 7 is coiled around keratin 19, corresponding exposed positions would not allow simultaneous binding of the two antibodies for steric reasons. This is further exemplified when e.g. amino acids 271-291 on keratin 19 pair up in the coiled coil with amino acids 283-303 on keratin 7. If an antibody to keratin 7 binds within the sequence of 283-303 on keratin 7, then an antibody to keratin 19 would not be able to bind to amino acids 271-291 on keratin 19 at the same time in the heterotypic complex because of steric hindrance.

A number of available antibodies that bind specifically to keratin 7 may be used in the methods presented herein, such as e.g. C-35, C-62, C-68, C18, C35, KS 7.18, LDS-68, LP1K, RCK105. However one antibody that is advantageously used in the methods presented herein is the keratin 7 monoclonal antibody produced by clone Ks 7.18 which is available from Progen Biotechnik, GmBH, Germany. Monoclonal antibody KS 7.18 binds specifically to amino acids 300-350 of the K7 moiety of the heterotypic complex of keratin 7 with keratin 19, and/or fragment(s) thereof.

Examples of available antibodies that will bind specifically to keratin 19 are A53-B/A2.26 aka Ks19.1, BM-19.21, CCD003, CCD004,CKS04, CKS06, SA21, SA 45, Ks19.2, LP2K. However one antibody that is advantageously used in the present method is the Ks 19.2 monoclonal antibody which is available from Progen Biotechnik, GmBH also known as the BM-19.21 monoclonal antibody produced by clone BM-19.21 which is available from Roche Diagnostics. BM-19.21 binds specifically to amino acids 346-367 of the of the K19 moiety of the heterotypic complex of keratin 7 with keratin 19, and/or fragment(s) thereof. Several of the antibodies against keratin 19 are described in: Epitope Specificity of 30 Monoklonal Antibodies against Cytokeratin Antigens: The ISOBM TD-1 workshop, Tumor Biol 1998;19:132-152.

Advantageously, each of the first and second primary antibodies or antigen-binding fragments, or variants, fusions or derivatives thereof are provided in a composition as an antibody cocktail, in aqueous form or in a freeze dried powder form. For the latter, a rehydration step is required to put the antibodies in a usable liquid form before use.

Alternatively the first and second primary antibodies are provided separately, i.e. one primary antibody is provided before the other when used in the methods presented herein. In one embodiment the first primary antibody recognizing a keratin 7 peptide and/or fragment(s) thereof is provided to a sample containing heterotypic complex of keratin 7 with keratin 19, and/or fragment(s) thereof. The first primary antibody will bind to an antigenic site present on the K7 moiety of the heterotypic complex of keratin 7 with keratin 19, and/or fragment(s) thereof and form an antigen - K7-antibody complex. Thereafter, the second primary antibody recognizing a keratin 19 peptide and/or fragment(s) thereof is provided to the sample containing heterotypic complex of keratin 7 with keratin 19 - K7-antibody. Said second primary antibody will bind to an antigenic site present on the K19 moiety of the heterotypic complex of keratin 7 with keratin 19 and form a K19-antibody - antigen - K7-antibody complex.

In an alternative embodiment, the second primary antibody recognizing a keratin 19 peptide and/or fragment(s) thereof is provided to a sample containing heterotypic complex of keratin 7 with keratin 19, and/or fragment(s) thereof. The second primary antibody will bind to an antigenic site present on the K19 moiety of the heterotypic complex of keratin 7 with keratin 19 and form an antigen - K19-antibody complex. Thereafter, the first primary antibody recognizing a keratin 7 peptide and/or fragment(s) thereof is provided to the sample containing heterotypic complex of keratin 7 with keratin 19 - K19-antibody. Said first primary antibody will bind to an antigenic site present on the K7 moiety of the heterotypic complex of keratin 7 with keratin 19 and form a K7-antibody - antigen - K19-antibody complex

The antibodies may be whole antibodies or fragments thereof, e.g. antigen-binding fragment, or variant, fusion or derivative thereof as long as they are capable of binding to the desired protein in vitro. Such binding specificity may be determined by methods well known in the art, such as e.g. ELISA, EIA, immunohistochemistry, immunoprecipitation, Western blots, chromatography and flow cytometry using transfected cells expressing all of the subunit or a heterodimer thereof (see Examples).

The term "antibody" includes substantially intact antibody molecules of any species such as rodents, e.g. murine, rat, guinea pig, or non-rodents such as rabbit, goat, sheep, dog, pig, camel, dromedary, donkey, horse or chicken, as well as chimaeric antibodies, humanized antibodies, human antibodies (wherein at least one amino acid is mutated relative to the naturally occurring human antibodies), single chain antibodies, bispecific antibodies, antibody heavy chains, antibody light chains, homo-dimers and heterodimers of antibody heavy and/or light chains, and antigen binding fragments and derivatives of the same. For example, the antibody may be a monoclonal antibody.

Antigenic specificity is conferred by variable domains and is independent of the constant domains, as known from experiments involving the bacterial expression of antibody fragments, all containing one or more variable domains. These molecules include Fab-like molecules; Fv molecules; single-chain Fv (ScFv) molecules where the V H and V L partner domains are linked via a flexible oligopeptide;) and single domain antibodies (dAbs) comprising isolated V domains. A general review of the techniques involved in the synthesis of antibody fragments which retain their specific binding sites is to be found in Winter & Milstein (1991) Nature 349, 293- 299.

Thus, by "antigen-binding fragment" is meant a functional fragment of an antibody that is capable of binding to any of the proteins K7or K19 or fragments thereof, and heterotypic complex of keratin 7 with keratin 19, and/or fragment(s) thereof. Exemplary antigen-binding fragments may be selected from the group consisting of Fv fragments (e.g. single chain Fv and disulphide-bonded Fv), Fab-like fragments (e.g. Fab fragments, Fab' fragments and F(ab) 2 fragments), single antibody chains (e.g. heavy or light chains), single variable domains (e.g. VH and VL domains) and domain antibodies (dAbs, including single and dual formats; i.e. dAb-linker-dAb).

Thus, in one embodiment the antibody or antigen-binding fragment, or variant, fusion or derivative thereof, comprises or consists of an intact antibody. In one embodiment, the antibody is a monoclonal antibody.

For example, the antibody or antigen-binding fragment, or a variant, fusion or derivative thereof, may consist essentially of an intact antibody. By "consist essentially of we mean that the antibody or antigen-binding fragment, variant, fusion or derivative thereof consists of a portion of an intact antibody sufficient to retain binding specificity for any of the two different proteins K7 and K19 or fragments thereof. In further embodiments, the two different proteins K7 and K19 are of human origin.

The term 'antibody' also includes all classes of antibodies, including IgG, IgA, IgM, IgD and IgE. In one embodiment, however, the antibody is an IgG molecule, such as an IgGI, IgG2, IgG3, or IgG4 molecule. In one embodiment, the antibody is an IgGI molecule. In a further embodiment, the antibody is an IgGI molecule with a kappa light chain.

In a further embodiment, the antibody may be a non-naturally occurring antibody. Of course, where the antibody is a naturally occurring antibody, it is provided in an isolated form (i.e. distinct from that in which it is found in nature).

Also included within the scope of the disclosure are modified versions of antibodies and antigen-binding fragments thereof, e.g. modified by the covalent attachment of polyethylene glycol or other suitable polymer, and uses of the same.

Methods of generating antibodies and antibody fragments are well known in the art. For example, antibodies may be generated via any one of several methods which employ induction of in vivo production of antibody molecules, screening of immunoglobulin libraries or generation of monoclonal antibody molecules by cell lines in culture. These include, but are not limited to, the hybridoma technology, the human B-cell hybridoma technology, and the Epstein-Barr virus (EBV)- hybridoma technology.

For example, generating monoclonal or polyclonal antibodies to K7, or K19 may be done by immunization where the whole protein or a suitable fragment thereof can be injected into non-human mammals (such as mice or rabbits), followed by boost injections, to produce an antibody response. Serum isolated from immunized animals may be isolated for the polyclonal antibodies contained therein, or spleens from immunized animals may be used for the production of hybridomas and monoclonal antibodies.

In one example, a monoclonal antibody to one of the proteins can be prepared from murine hybridomas according to the classical method of Kohler and Milstein {Nature, 256:495, 1975) or derivative methods thereof. Briefly, a mouse (such as Balb/c) is repetitively inoculated with a few micrograms of the selected protein or peptide fragment thereof or a suitable carrier conjugate thereof over a period of a few weeks. The mouse is then sacrificed, and the antibody-producing cells of the spleen isolated. The spleen cells are fused by means of polyethylene glycol with mouse myeloma cells, and the excess un-fused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall (Enzymol., 70:419, 1980), and derivative methods thereof.

Selected positive clones can be expanded and their monoclonal antibody product harvested for use.

Commercial sources of antibodies include DAKO A/S, Abeam, Lab Vision, BioCare Medical, Cell Marque Corp. Abnova, Acris, Progen, Santa cruz biotech etc.

Polyclonal antibody-producing animals are identified by bleeding immunized animals and selection of appropriate animal with a suitable polyclonal antibody-titer thereof.

In some embodiments, antibodies are purified before use. Purification of antibodies are done using techniques available in the art and known to the skilled person.

Generation of antibodies K7 and K19 are described in the art and available from commercial sources as described herein, or being available using techniques known to a skilled artisan using references enclosed herein.

The antibody or antigen-binding fragment or derivative thereof may also be produced by recombinant means. Suitable monoclonal antibodies to selected antigens and proteins may be prepared by techniques known to a skilled person. Antibody fragments can also be obtained using methods well known in the art. For example, antibody fragments may be prepared by proteolytic hydrolysis of the antibody or by expression in E. coli or mammalian cells (e.g. Chinese hamster ovary cell culture or other protein expression systems) of DNA encoding the fragment. Alternatively, antibody fragments can be obtained by pepsin or papain digestion of whole antibodies by conventional methods. Thus, in one embodiment at least one of the primary antibodies used in the methods as presented herein is a monoclonal antibody. In a further embodiment at least one of the primary antibodies used in the methods is a recombinant antibody. Alternatively, complex formation may be detected using duolink (O-link bioscience). Conventional immunofluorescence can reveal the presence of the individual proteins, but not the formation of heterodimers between different proteins. Using Duolink it would be possible to locate exactly where hetero dimerization takes place and quantify the relative extent of the interaction. Thus, it would be possible using the duolink technique to locate and quantify the heterotypic complex between keratin 7 and keratin 19 by using one antibody specific for keratin 7 and one antibody specific for keratin 19 wherein both antibodies bind simultaneously to the heterotypic complex between keratin 7 and keratin 19.

The antibodies or antigen-binding fragments or derivatives thereof, or composition comprising said antibodies or antigen-binding fragments or derivatives thereof described herein may be lyophilized for storage and reconstituted in a suitable carrier prior to use. Any suitable lyophilisation method (e.g. spray drying, cake drying) and/or reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate. In one embodiment, the lyophilized (freeze dried) composition loses no more than about 20%, or no more than about 25%, or no more than about 30%, or no more than about 35%, or no more than about 40%, or no more than about 45%, or no more than about 50% of its activity (prior to lyophilisation) when re-hydrated. It will be further appreciated by persons skilled in the art that the antibodies and antigen-binding fragments, variants, fusions and derivatives thereof, described herein may exist in monomeric form or in the form of a homo-or hetero- multimer thereof (e.g. dimer, trimer, tetramer, pentamer, etc.).

Further provided herein is that the primary antibodies or fragments thereof may be labelled directly or indirectly, with a detectable moiety. By directly labeled is meant that the detectable moiety is attached to the antibody. By indirect labeled it is meant that the detectable moiety is attached to a linker, such as, for example, a secondary or tertiary antibody. The detectable moiety may be any moiety or marker known to those skilled in the art, or as described herein, and as being such a moiety being capable of generating a signal that allows the direct or indirect quantitative or relative measurement of a molecule to which it is attached.

A wide variety of detectable moieties, or labels, and conjugation techniques are known and reported extensively in both the scientific and patent literature. Suitable labels include radionuclides, enzymes, substrates, cofactors, inhibitors, fluorescent agents, chemiluminescent agents, magnetic particles and the like which are well-known to the skilled person. The detectable moiety may be a single atom or molecule which is either directly or indirectly involved in the production of a detectable species. Optionally, the detectable moiety is selected from the group consisting of a fluorescent moiety, an enzyme linked moiety, a biotinylated moiety and a radiolabeled moiety, as described further herein, e.g. below. By "label", "detectable moiety" is meant any detectable tag that can be attached directly (e.g., a fluorescent molecule integrated into a polypeptide) or indirectly (e.g., by way of binding to a primary antibody with a secondary, tertiary or further antibody with an integrated fluorescent molecule) to the molecule of interest. Thus, a label, marker or detectable moiety is any tag that can be visualized, for example, with imaging methods.

By a "detectable moiety" we further include the meaning that the moiety is one which, when located at the target site following providing the composition to a biological sample, such as a tissue sample, or a biological fluid sample, may be detected in vitro. That includes that the detectable moiety is signal generating and it is further convenient and thus included in further embodiments if the detectable moiety may be detected and the relative amount and/or location of the moiety (for example, the location on an tissue sample) may be determined. Detectable moieties are well known in the art. Thus, antibodies or antigen-binding fragments or derivatives thereof, or the composition comprising such antibodies or antigen-binding fragments or derivatives thereof are useful in methods further exemplified herein by methods and uses for detection of heterotypic complex of keratin 7 with keratin 19, and/or fragment(s) thereof, diagnosis or prognosis malignant neoplastic disease such as e.g. lung cancer, bladder cancer, esophagus cancer and ovarian cancer in vitro of biological samples. In further embodiments, immunochemical methods used are exemplified herein.

Suitable detectable moieties are well known in the art and the attachment or linking of these moieties to polypeptides and proteins is further well known in the art. Further examples of detectable moieties are an enzyme; an enzyme substrate; an enzyme inhibitor; coenzyme; enzyme precursor; apoenzyme; fluorescent substance; pigment; chemiluminescent compound; luminescent substance; coloring substance; magnetic substance; or a metal particle such as gold colloid; a radioactive substance such as ¹²⁵I, ¹³¹I, ³²P, ³H, ³⁵S, or ¹⁴C; a phosphorylated phenol derivative such as a nitrophenyl phosphate, luciferin derivative, or dioxetane derivative; or the like. The enzyme may be a dehydrogenase; an oxidoreductase such as a reductase or oxidase; a transferase that catalyzes the transfer of functional groups, such as an amino; carboxyl, methyl, acyl, or phosphate group; a hydrolase that may hydrolyze a bond such as ester, glycoside, ether, or peptide bond; a lyase; an isomerase; or a ligase. The enzyme may also be conjugated to another enzyme. The enzyme may be detected by enzymatic cycling. For example, when the detectable label is an alkaline phosphatase, a measurement may be made by observing the fluorescence or luminescence generated from a suitable substrate, such as an umbelliferone derivative. The umbelliferone derivative may comprise 4-methyl- umbellipheryl phosphate. The fluorescent or chemiluminescent label may be a fluorescein isothiocyanate; a rhodamine derivative such as rhodamine B isothiocyanate or tetramethyl rhodamine isothiocyanate; a dancyl chloride (5-(dimethylamino)-1-naphtalenesulfonyl chloride); a dancyl fluoride; a fluorescamine (4-phenylspiro[furan-2(3H); ly-(3yH)-isobenzofuran]- 3;3y-dione); a phycobiliprotein such as a phycocyanine or phycoerythrin; an acridinium salt; a luminol compound such as lumiferin, luciferase, or aequorin; imidazoles; an oxalic acid ester; a chelate compound of rare earth elements such as europium (Eu), terbium (Tb) or samarium (Sm); or a coumarin derivative such as 7-amino-4-methylcoumarin. The label may also be a hapten, such as adamantine, fluoroscein isothiocyanate, or carbazole. The hapten may allow the formation of an aggregate when contacted with a multi-valent antibody or (streptavidin containing moiety. Further examples of detectable moieties include, but are not limited to, the following: radioisotopes (e.g. ³H, ¹⁴C, ³⁵S, ¹²³I, ¹²⁵I, ¹³¹I, ⁹⁹Tc, ¹¹¹In, ⁹⁰Y, ¹⁸⁸Re), radionuclides (e.g. 11C, 18F, 64Cu), fluorescent labels (e.g. FITC, rhodamine, lanthanide phosphors, carbocyanine), enzymatic labels (e.g. horseradish peroxidase, [beta]-galactosidase, luciferase, alkaline phosphatase), chemiluminescent, biotinyl groups and predetermined polypeptide epitopes recognized by a secondary binding entity (e.g. leucine zipper pair sequences, binding sites for secondary antibodies, metal binding domains, epitope or protein tags, carbohydrates). In some embodiments, labels are attached by spacer arms of various lengths to reduce potential steric hindrance.

In indirect labelling, an additional molecule or moiety is brought into contact with, or generated at the site of, the antibody-antigen complexes, i.e. immune-complexes, between the primary antibody and the protein it binds to. For example, a detectable moiety such as an enzyme can be attached to or associated with the detecting antibody or detecting molecule as exemplified herein. The signal-generating molecule can then generate a detectable signal at the site of the immune-complex. For example, an enzyme, when supplied with suitable substrate, can produce a visible or detectable product at the site of the immune-complex.

As another example of indirect labelling, an additional molecule (which can be referred to as a binding agent) that can bind to either the molecule of interest or to the antibody (primary antibody) of interest, such as a second antibody to the primary antibody, can be contacted with the immunocomplex. The additional molecule can have signal-generating molecule or detectable moiety.

The additional molecule may be an antibody, which can thus be termed a secondary, tertiary or further antibody. Binding of a secondary antibody to the primary antibody can form a so- called sandwich with the first (or primary) antibody and the molecule of interest. The immune- complexes can be contacted with the labelled, secondary antibody under conditions effective and for a period of time sufficient to allow the formation of secondary immune complexes. The secondary immune complexes can then be generally washed to remove any non-specifically bound labelled secondary antibodies, and the remaining label in the secondary immune complexes can then be detected. The additional molecule can also be or include one of a pair of molecules or moieties that can bind to each other, such as the biotin/avidin molecules, and the detecting antibody or detecting molecule should then include the other member of the pair.

Further examples of indirect labelling include the detection of primary antibody-antigen (immune-complexes) by a two-step approach. For example, a molecule (which can be referred to as a first binding agent), such as an antibody, that has binding affinity for the primary immune complex between the primary antibody-antigen complex can be used to form secondary complexes. After washing, the secondary complex can be contacted with another further molecule (which can be referred to as a second binding agent) that has binding affinity for the first binding agent, again under conditions effective and for a period of time sufficient to allow the formation of tertiary complexes. In this example the second binding agent may be linked to a detectable moiety, allowing detection of the tertiary complexes thus formed. This system may further comprise means to provide for signal amplification.

Other examples of primary, secondary or further binding agents with means for signal amplification are conjugated anti-immunoglobulins such as biotinylated antibodies (e.g., conjugated with avidin/ streptavidin) or staphylococcal Protein A (binds IgG), Protein G, dextran, aptamers, proteins, peptides, small organic molecules, natural compounds (e.g. steroids), non-peptide polymers, or any other molecules that specifically and efficiently bind to other molecules conjugated with a detectable moiety of not. In one further embodiment, a secondary, tertiary or further binding agent is an antibody such as an anti-mouse conjugate, e.g. a swine anti-mouse antibody. The conjugate may be a conjugate to dextrane, HRP, biotin, alkali phosphatase, etc. as described supra.

In one embodiment the detectable moiety is a swine anti-mouse antibody conjugated with dextran and HRP.

In a further embodiment a secondary, tertiary or further binding agent is an antibody such as an anti-rabbit conjugate, e.g. a goat anti-rabbit conjugate. The conjugate may be a conjugate to dextrane, HRP, biotin, etc. described supra.

In one embodiment, the detectable moiety is a goat anti-rabbit conjugated with dextran. In a further embodiment a secondary, tertiary or further binding agent is an antibody such as an anti-goat conjugate, such as e.g. a rabbit anti-goat conjugate. The conjugate may be a conjugate to dextrane, HRP1 biotin, etc. described supra.

In one embodiment the detectable moiety is rabbit anti-goat conjugated with dextran and Alkaline phosphatase, AP.

In still a further embodiment, the composition provided herein further comprises a buffer. Examples of buffers are Tris-buffers such as Tris-HCI, and PBS-buffers. Suitable buffers are available and known in the art.

Further additives to the buffers may be e.g. Tween®20, BSA, sodium azide, glycerol, and water, and a pH from about 5.5 to about 8.0, such as about 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 or 8.0.

The antibody composition may when in a liquid form be provided in a "ready-to-use" form or in a concentrated form which may be diluted before use in any appropriate buffer system upon use, for example at least 1x10, 1x20, 1x30, 1x40, 1x50, 1x60, 1x70, 1x80, 1x90, 1x100, 1x1000, 1x10000 and all ranges and values there between such as in e.g. the buffer systems provided here in or ant that may be apparent to a person skilled in the art.

In one advantageous embodiment the first primary antibody monoclonal antibody Ks 7.18 recognizing keratin 7 peptide and/or fragment(s) thereof is conjugated to biotin, and the second primary antibody BM 19.21 recognizing a keratin 19 peptide and/or fragment(s) thereof is conjugated to peroxidase (horse radish peroxidase (HRP)).The conjugation of biotin or HRP is known to a person skilled in the art.

The composition may also be used alone or in combination with other means for detecting malignant neoplasm, including, but not limited to means for detecting and measuring another tumor markers such as: CA125, HE4, CA 19-9, CEA, CYFRA 21-1, SCC, ProGRP, CA242, PSA or diagnostic means such as ultrasound, CT-scans, chest radiography and/or IHC markers such as K5,K20,TTF-1, CDX2, MUC 2, p63, beta-catenin, p53, 34-beta-E12, ER, CD56, NCAM, K6, K7, K19.

### Methods and uses of the composition

The herein described antibodies or antigen-binding fragments or derivatives thereof, or the composition comprising such antibodies or antigen-binding fragments or derivatives thereof may be used in various immuno-methods, such as immunohistochemical (IHC) and immunochemical methods. General protocols for such immuno-methods, particularly immunohistochemistry methods, are known in the art. In addition, methods and uses may be combined with other diagnostic methods to improve the outcome of the differential diagnosis.

The importance of accurately determining the presence or absence of malignant neoplasms is evident. The impact on both the patient and health care system is further also evident. Thus, some embodiments of the methods and uses provide detecting of heterotypic complex of keratin 7 with keratin 19, and/or fragment(s) thereof within a given biological sample. The methods and uses comprises obtaining a biological sample from a subject, contacting said sample with the antibodies or antigen-binding fragments or derivatives thereof, or the composition comprising such antibodies or antigen-binding fragments or derivatives thereof disclosed herein specific for the two proteins K7 and K19, detecting an interaction between said antibodies and heterotypic complex of keratin 7 with keratin 19, or fragment(s) thereof, wherein the detection of an interaction indicates the presence or absence of heterotypic complex of keratin 7 with keratin 19, and/or fragment(s) thereof, thereby allowing for e.g. detection of heterotypic complex of keratin 7 with keratin 19, and/or fragment(s) thereof, detection of malignant neoplastic disease in a biological sample, diagnosis, prognosis etc. according to any of the methods disclosed herein, the results of which one may determine if a subject is healthy, or is having a malignant neoplastic disease.

The composition and methods of the invention can be used when the malignant neoplastic disease is bile duct cancer (extrahepatic), bladder cancer, breast cancer, carcinoma of unknown (primary), cervical cancer, colon cancer, endometrial cancer, esophageal cancer, gallbladder cancer, gastric (stomach) cancer, head and neck cancer, hepatocellular (liver) cancer, hypopharyngeal cancer, kidney cancer, laryngeal cancer, liver cancer (primary), lung cancer (non-small cell), lung cancer (small cell), mesothelioma, non-small cell lung cancer, ovarian cancer, ovarian epithelial cancer (surface epithelial-stromal tumor), ovarian germ cell tumor, ovarian low malignant potential tumor, pancreatic cancer, prostate cancer, rectal cancer, renal cell carcinoma (kidney cancer), salivary gland cancer, small cell lung cancer, small intestine cancer, stomach cancer, testicular cancer, thyroid cancer, transitional cell cancer of the renal pelvis and ureter, or uterine cancer (endometrial).

The composition and methods of the invention are suitable when the malignant neoplastic disease is lung cancer, bladder cancer, esophagus cancer, hepatocellular cancer, pancreatic cancer, gastric cancer and ovarian cancer.

The composition and methods of the invention are particularly suitable when the malignant neoplastic disease is one of lung cancer, bladder cancer, esophagus cancer or ovarian cancer.

Benign disease tends to raise tumor marker values a bit for these individuals in relation to healthy individuals. Typical benign diseases elevating the marker concentration are diseases of the biliary tract, liver and kidney. Due to these elevated values a border line zone or a "gray zone" is frequently used for most markers and this grey zone also renders the markers unsuitable for diagnostic purposes. Tumor marker values in the borderline zone are very uncertain and cannot be classified as either tumor or benign disease. The subjects falling within this gray zone may be falsely suspected as having or not having cancer. Subjects falling within this gray zone will benefit from the methods provided herein. Within the disclosed methods and uses of heterotypic complex of keratin 7 with keratin 19, and/or fragment(s) thereof may be present at elevated levels, at decreased levels, or altogether absent within a sample taken from a subject in a particular clinical state (e.g., healthy or having a malignant neoplastic disease such as e.g. lung cancer, bladder cancer, esophagus cancer or ovarian cancer).

Accordingly, differential presence of the heterotypic complex of keratin 7 with keratin 19, and/or fragment(s) thereof found in a given biological sample provides useful information regarding a probability of whether a subject being tested has malignant neoplastic diseases such as e.g. lung cancer, bladder cancer, esophagus cancer and ovarian cancer or is healthy. A probability that a subject being tested has a malignant neoplastic diseases such as e.g. lung cancer, bladder cancer, esophagus cancer and ovarian cancer or is healthy depends on whether the quantity of heterotypic complex of keratin 7 with keratin 19, and/or fragment(s) thereof in a test sample taken from said subject is statistically significant from a quantity of heterotypic complex of keratin 7 with keratin 19, and/or fragment(s) thereof in a biological sample taken from healthy subjects or a control level known to exist in healthy subjects.

A difference in heterotypic complex of keratin 7 with keratin 19, and/or fragment(s) thereof found in a given biological sample may also be used to determine whether a subject known to have a malignant neoplastic disease such as e.g. lung cancer, bladder cancer, esophagus cancer or ovarian cancer is responding to a therapeutic treatment being administered. A quantity of the heterotypic complex of keratin 7 with keratin 19, and/or fragment(s) thereof detected in a sample taken at time of therapy is compared to a quantity of the heterotypic complex of keratin 7 with keratin 19, and/or fragment(s) thereof detected in a sample taken prior to an administration of treatment. In addition, a quantity of the heterotypic complex of keratin 7 with keratin 19, and/or fragment(s) thereof detected in a sample taken at time of therapy is compared to a reference of the heterotypic complex of keratin 7 with keratin 19, and/or fragment(s) thereof indicative of a healthy subject. Based on a comparison, one can determine whether said subject is responding to a therapeutic treatment, and to what degree the response is.

Furthermore, a difference in presence of the heterotypic complex of keratin 7 with keratin 19, and/or fragment(s) thereof found in a given biological sample may also be used to determine whether a subject known to have a malignant neoplastic disease such as e.g. lung cancer, bladder cancer, esophagus cancer or ovarian cancer will respond to a given therapeutic treatment. A quantity of the heterotypic complex of keratin 7 with keratin 19, and/or fragment(s) thereof detected in a sample taken from a subject diagnosed as having a malignant neoplastic disease such as e.g. lung cancer, bladder cancer, esophagus cancer or ovarian cancer is compared to reference panels of the heterotypic complex of keratin 7 with keratin 19, and/or fragment(s) thereof taken from subjects with similar diagnoses that have undergone different forms of treatment. Reference panels of the heterotypic complex of keratin 7 with keratin 19, and/or fragment(s) thereof generated from samples taken from subjects exposed to a given treatment, wherein the treatment resulted in a positive outcome are considered to indicate that the given treatment had a positive effect on the subject and therefore would be deemed successful. Reference panels of the heterotypic complex of keratin 7 with keratin 19, and/or fragment(s) thereof generated from samples taken from subjects exposed to a given treatment, wherein the treatment resulted in a neutral outcome are considered to indicate that the given treatment had no therapeutic effect on the subject and would therefore be deemed unsuccessful. Reference panels of the heterotypic complex of keratin 7 with keratin 19, and/or fragment(s) thereof generated from samples taken from subjects exposed to a given treatment, wherein the treatment resulted in a negative outcome are considered to indicate that the given treatment had no therapeutic effect on the subject and would be deemed unsuccessful. Based on the comparison, one skilled in the art would be able to administer the best mode of treatment for said subject. Additionally, differential presence of the heterotypic complex of keratin 7 with keratin 19, and/or fragment(s) thereof found in a given biological sample may also be used to determine the stage of malignant neoplastic disease such as e.g. lung cancer, bladder cancer, esophagus cancer or ovarian cancer in a subject.

A quantity of the heterotypic complex of keratin 7 with keratin19 detected in a sample taken from a subject diagnosed as having a malignant neoplastic disease such as e.g. lung cancer, bladder cancer, esophagus cancer or ovarian cancer is compared to reference biomarker panel taken from subjects known to have a specific stage or grade of malignant neoplastic disease such as e.g. lung cancer, bladder cancer, esophagus cancer or ovarian cancer. Based on the comparison, one would be able to determine the stage or grade at which the malignant neoplastic disease such as e.g. lung cancer, bladder cancer, esophagus cancer or ovarian cancer within said subject.

The heterotypic complex of keratin 7 with keratin 19, and/or fragment(s) thereof may be present at an elevated level, at a decreased level, or altogether absent within a sample taken from a subject in a particular clinical state (e.g., healthy or having malignant neoplastic disease such as e.g. lung cancer, bladder cancer, esophagus cancer or ovarian cancer).

Accordingly, any presence of the heterotypic complex of keratin 7 with keratin 19, and/or fragment(s) thereof found in a given biological sample provides useful information regarding a probability of whether a subject being tested has a malignant neoplastic disease such as e.g. lung cancer, bladder cancer, esophagus cancer or ovarian cancer or is healthy. A probability that a subject being tested has malignant neoplastic disease such as e.g. lung cancer, bladder cancer, esophagus cancer or ovarian cancer or is healthy depends on whether the quantity of the heterotypic complex of keratin 7 with keratin 19, and/or fragment(s) thereof in a test sample taken from said subject is statistically significant from a quantity of the heterotypic complex of keratin 7 with keratin 19, and/or fragment(s) thereof in a biological sample taken from healthy subjects or a control level known to exist in healthy subjects.

A subject that is said to have malignant neoplastic disease such as e.g. lung cancer, bladder cancer, esophagus cancer or ovarian cancer possesses morphological, biochemical, and functional alterations of their tissue such that the tissue can be characterized as a malignant neoplastic disease such as e.g. lung cancer, bladder cancer, esophagus cancer or ovarian cancer. The stage to which a malignant neoplastic disease such as e.g. lung cancer, bladder cancer, esophagus cancer or ovarian cancer has progressed can be determined using known methods currently available and presented herein.

Data analysis to analyze the presence or absence of the heterotypic complex of keratin 7 with keratin 19, and/or fragment(s) thereof may include the steps of determining signal strength (e.g., intensity of peaks) of the detected biomarker and removing "outliers" (data deviating from a predetermined statistical distribution). An example is the normalization of peaks, a process whereby the intensity of each peak relative to some reference is calculated. For example, a reference can be background noise generated by an instrument and/or a chemical (e.g., energy absorbing molecule), which is set as zero in the scale. Then the signal strength detected for each protein can be displayed in the form of relative intensities in the scale desired (e.g., 100). In an embodiment, an observed signal for a given peak can be expressed as a ratio of the intensity of that peak over the sum of the entire observed signal for both peaks and background noise in a specified mass to charge ratio range. In an embodiment, a standard may be admitted with a sample so that a peak from the standard can be used as a reference to calculate relative intensities of the signals observed for the heterotypic complex of keratin 7 with keratin 19, and/or fragment(s) thereof detected.

The resulting data can be transformed into various formats for displaying, typically through the use of computer algorithms. Using any of the above display formats, it can be readily determined from a signal display whether the heterotypic complex of keratin 7 with keratin 19, and/or fragment(s) thereof is detected in a sample.

Exemplary method may be used to e.g. diagnose, prognose, staging of malignant neoplastic disease such as e.g. lung cancer, bladder cancer, esophagus cancer or ovarian cancer, predict treatment outcome, predict the likelihood of malignant neoplastic disease such as e.g. lung cancer, bladder cancer, esophagus cancer or ovarian cancer recurrence etc. as further described herein.

Recurrence means the malignant neoplastic disease such as e.g. lung cancer, bladder cancer, esophagus cancer or ovarian cancer has returned after an initial (or subsequent) treatment(s). Representative initial treatments include radiation treatment, chemotherapy, anti- hormone treatment and/or surgery.

Some methods disclosed herein are useful for prognosis of malignant neoplastic disease such as e.g. lung cancer, bladder cancer, esophagus cancer or ovarian cancer. Prognosis is the likely outcome of the disease (typically independent of treatment). The methods disclosed herein may be used to prognose, i.e. to predict a likely outcome of the diseases such as e.g. a recurrence in a sample collected well prior to such recurrence. A poor (or poorer) prognosis is likely for a subject with a more aggressive cancer. In some method embodiments, a poor prognosis is less than 5 year survival (such as less than 1 year survival or less than 2 year survival) of the patient after initial diagnosis of the neoplastic disease. In some method embodiments, a good prognosis is greater than 2-year survival (such as greater than 3-year survival, greater than 5-year survival, or greater than 7-year survival) of the patient after initial diagnosis of the neoplastic disease.

Still other method embodiments predict treatment outcome of malignant neoplastic diseases such as e.g. lung cancer, bladder cancer, esophagus cancer or ovarian cancer in patients, and are useful for directing (e.g., selecting useful) treatment modalities for cancer patients. As discussed elsewhere in this specification, expression of the heterotypic complex of keratin 7 with keratin 19, and/or fragment(s) thereof predicts that treatment (e.g. immunotherapy, thermotherapy, laser therapy, surgery, radiation therapy, chemotherapy) is likely to fail (e.g., the disease will recur). Hence, the heterotypic complex of keratin 7 with keratin 19, and/or fragment(s) thereof can be used by caregivers to counsel cancer patients as to the likely success of treatment (e.g. immunotherapy, laser therapy, surgery, thermotherapy, radiation therapy, chemotherapy). Taken in the context of the particular subject's medical history, the patient and the caregiver can make better informed decisions of whether or not to treat (e.g., perform surgery) and/or whether or not to provide alternate treatment (such as, external beam radiotherapy, brachytherapy, chemotherapy, or watchful waiting).

The present disclosure thus relates to methods for diagnosis and prognosis of malignant neoplastic disease such as e.g. lung cancer, bladder cancer, esophagus cancer or ovarian cancer by detecting the heterotypic complex of keratin 7 with keratin 19, and/or fragment(s) thereof expressed within a biological sample of a given subject, wherein the presence or absence of the heterotypic complex of keratin 7 with keratin 19, and/or fragment(s) thereof allows for the diagnosis or prognosis of a subject as healthy or having a malignant neoplastic disease such as e.g. lung cancer, bladder cancer, esophagus cancer or ovarian cancer. In one embodiment, the methods detect the presence of heterotypic complex of keratin 7 with keratin 19, and/or fragment(s) thereof in a sample wherein the marker is not expressed in healthy, disease-free individuals. In related embodiments, the methods of the invention detect elevated levels of heterotypic complex of keratin 7 with keratin 19, and/or fragment(s) thereof that is present at a higher level in samples from individuals that have malignant neoplastic disease such as e.g. lung cancer, bladder cancer, esophagus cancer or ovarian cancer, as compared to normal, healthy individuals.

One aspect of the present invention is an *in vitro* method for detecting heterotypic complex of keratin 7 with keratin 19, and/or fragment(s) thereof in a biological sample, the method comprising the steps of
a) contacting said biological sample with a composition comprising at least two antibodies, antigen-binding fragment(s), or combinations thereof, wherein at least one first antibody, antigen-binding fragment, or combination thereof recognizes a sequence located within the 256-412 amino acid sequence, or more preferably within the 300-380 amino acid sequence in the keratin 7 moiety of the heterotypic complex of keratin 7 with keratin 19 and/or fragment(s) thereof, and at least one second antibody, antigen-binding fragment, or combination thereof recognizes a sequence located within the 244-400 amino acid sequence, or more preferably within the 311-375 amino acid sequence in the keratin 19 moiety of the heterotypic complex of keratin 7 with keratin 19 and/or fragment(s) thereof, said first and second antibodies, antigen-binding fragment(s), or combination thereof-are capable of binding specifically and simultaneously to heterotypic complex of keratin 7 with keratin 19 and/or fragment(s) thereof; or alternatively
b) contacting said biological sample with a first antibody, antigen-binding fragment, or combination thereof recognizes a sequence located within the 256-412 amino acid sequence, or more preferably within the 300-380 amino acid sequence in the keratin 7 moiety of the heterotypic complex of keratin 7 with keratin 19 and/or fragment(s) thereof; and
c) contacting said biological sample with a second antibody, antigen-binding fragment, or combination thereof recognizes a sequence located within the 244-400 amino acid sequence, or more preferably within the 311-375 amino acid sequence in the keratin 19 moiety of the heterotypic complex of keratin 7 with keratin 19 and/or fragment(s) thereof; and
d) detecting heterotypic complex of keratin 7 with keratin 19, and/or fragment(s) thereof, wherein steps b) and c) may be performed in any order or, simultaneously.

For example, in one embodiment the biological sample is contacted with a composition comprising at least two targeting agents, wherein at least one first targeting agent recognizes a keratin 7-peptide and/or fragment(s) thereof, and at least one second targeting agent recognizes a keratin 19-peptide and/or fragment(s) thereof. Said first and second targeting agents are capable of binding specifically and simultaneously to heterotypic complex of keratin 7 with keratin 19, and/or fragment(s) thereof. The specific and simultaneous binding of said first and second targeting agents to heterotypic complex of keratin 7 with keratin 19, and/or fragment(s) thereof is thereafter detected by means of any methods well known to persons skilled in the art, or, alternately by advantageous methods as described herein.

In an alternative embodiment the biological sample is not contacted with the first and second primary antibodies as part of a composition but the first and second primary antibodies are provided separately, i.e. one primary antibody is provided before the other. For example, in one embodiment the first primary antibody recognizing a keratin 7 peptide and/or fragment(s) thereof is contacted with a biological sample.The first primary antibody will bind to an antigenic site present on the K7 moiety of a heterotypic complex of keratin 7 with keratin 19, or fragment(s) thereof, present in said biological sample. A complex between the heterotypic complex of keratin 7 with keratin 19, and/or fragment(s) thereof and the antibody recognizing a keratin 7 peptide and/or fragment(s) thereof will form. Thereafter, the second primary antibody recognizing a keratin 19 peptide and/or fragment(s) thereof is provided to the biological sample containing heterotypic complex of keratin 7 with keratin 19, and/or fragment(s) thereof bound to the first primary antibody recognizing a keratin 7 peptide and/or fragment(s) thereof. Said second primary antibody will bind to an antigenic site present on the K19 moiety of the heterotypic complex of keratin 7 with keratin 19, and/or fragment(s) thereof and form a K19-antibody - antigen - K7-antibody complex, i.e. a triple complex comprising heterotypic complex of keratin 7 with keratin 19, and/or fragment(s) thereof and the two primary antibodies recognizing keratin 7 and keratin 19 peptides, and/or fragment(s) thereof, respectively.

In an alternative embodiment, the second primary antibody recognizing a keratin 19 peptide and/or fragment(s) thereof is provided to a biological sample.The second primary antibody will bind to an antigenic site present on the K19 moiety of the heterotypic complex of keratin 7 with keratin 19, and/or fragment(s) thereof and form an antigen - K19-antibody complex. Thereafter, the first primary antibody recognizing a keratin 7 peptide and/or fragment(s) thereof is provided to the sample containing heterotypic complex of keratin 7 with keratin 19, and/or fragment(s) thereof - K19-antibody. Said first primary antibody will bind to an antigenic site present on the K7 moiety of the heterotypic complex of keratin 7 with keratin 19, and/or fragment(s) thereof and form a K7-antibody - antigen - K19-antibody complex, i.e. a triple complex comprising heterotypic complex of keratin 7 with keratin 19, and/or fragment(s) thereof and the two primary antibodies recognizing keratin 7 and keratin 19 peptides, and/or fragment(s) thereof, respectively.

In a further step the amount of heterotypic complex of keratin 7 with keratin 19, and/or fragment(s) thereof detected may be compared to a positive and/or negative control, wherein the positive control comprises heterotypic complex of keratin 7 with keratin 19, and/or fragment(s) thereof and the negative control does not comprise heterotypic complex of keratin 7 with keratin 19, and/or fragment(s) thereof. In a further embodiment, the positive control comprises tissue or body fluids obtained from subjects diagnosed with malignant neoplastic disease, and the negative control is a biological sample obtained from healthy subjects who are not suffering from malignant disease.

Optionally, a scoring may be done of the detected antigen-antibody complexes according to a standard scoring system known in the art or described herein.

The sample may, of course, be any biological sample which possibly may comprise heterotypic complex of keratin 7 with keratin 19, and/or fragment(s) thereof. Examples of such biological samples are tissue samples, body fluid samples or cell samples from humans, rodents, such as mice, rats, guinea pigs, or from goats, sheep, pigs, camels, dogs, cats, and even rabbits or otherwise as disclosed herein. In one embodiment, the sample is from a human. In still a further embodiment, the sample is a tissue sample or a human body fluid sample, such as e.g. blood serum, blood plasma, lymph,, exudates, feces, gastric acid, gastric juice, lymph, mucus, pericardial fluid, peritoneal fluid, pleural fluid, pus, saliva, sputum, synovial fluid, tears, sweat, vaginal secretion, vomit and urine. The biological samples may need to be prepared in order to work, and pre-treatment of the sample may be done according to methods known to the person skilled in the art.

The *in vitro* method for detecting heterotypic complex of keratin 7 with keratin 19, and/or fragment(s) thereof in a biological sample as presented herein may advantageously be used for
i) diagnosing and/or prognosing malignant neoplastic disease in a subject, or
ii) predicting efficacy of treatment of malignant neoplastic disease in a subject, or
iii) assessing outcome of treatment of malignant neoplastic disease in a subject, or
iv) assessing recurrence of malignant neoplastic disease in a subject, wherein the subject is a mammal having, or is suspected of having, a malignant neoplastic disease.

The malignant neoplastic diseases may be any one of bile duct cancer (extrahepatic), bladder cancer, breast cancer, carcinoma of unknown (primary), cervical cancer, colon cancer, endometrial cancer, esophageal cancer, gallbladder cancer, gastric (stomach) cancer, head and neck cancer, hepatocellular (liver) cancer, hypopharyngeal cancer, kidney cancer, laryngeal cancer, liver cancer (primary), lung cancer (non-small cell), lung cancer (small cell), mesothelioma, non-small cell lung cancer, ovarian cancer, ovarian epithelial cancer (surface epithelial-stromal tumor), ovarian germ cell tumor, ovarian low malignant potential tumor, pancreatic cancer, prostate cancer, rectal cancer, renal cell carcinoma (kidney cancer), salivary gland cancer, small cell lung cancer, small intestine cancer, stomach cancer, testicular cancer, thyroid cancer, transitional cell cancer of the renal pelvis and ureter, uterine cancer (endometrial).

However, the methods as disclosed herein are useful for
i) diagnosing and/or prognosing malignant neoplastic disease in a subject, or
ii) predicting efficacy of treatment of malignant neoplastic disease in a subject, or
iii) assessing outcome of treatment of malignant neoplastic disease in a subject, or
iv) assessing recurrence of malignant neoplastic disease in a subject; when the subject is a mammal having, or is suspected of having, lung cancer, bladder cancer, esophagus cancer, hepatocellular cancer, pancreatic cancer, gastric cancer and ovarian cancer. The methods disclosed herein are particularly useful when the subject is a mammal having, or is suspected of having, lung cancer, bladder cancer, esophagus cancer, and ovarian cancer.

For example, one aspect of the disclosure is a method for detection in vitro of malignant neoplastic disease in a biological sample. The sample may be any biological sample possibly comprising malignant neoplastic disease, said method comprising the steps of
a) contacting said biological sample with a composition as described herein; or
b) contacting said biological sample with a first targeting agent recognizing a cytokeratin 7 peptide and/or fragment(s) thereof; and
c) contacting said biological sample with a second targeting agent recognizing a cytokeratin 19 peptide and/or fragment(s) thereof: and
d) detecting said Heterotypic complex of cytokeratin 7 with cytokeratin 19; wherein steps b) and c) may be performed in any order or, simultaneously.

The specific and simultaneous binding of said first and second targeting agents to heterotypic complex of keratin 7 with keratin 19, and/or fragment(s) thereof may be detected by means of any methods well known to persons skilled in the art, or, alternately by advantageous methods as described herein, thereby detecting the malignant neoplastic disease in the biological sample.

In a further step the amount of heterotypic complex of keratin 7 with keratin 19, and/or fragment(s) thereof detected may be compared to a positive and/or negative control, wherein the positive control comprises cells from a subject who is suffering from malignant neoplastic disease, and the negative control comprises cells from healthy subjects who are not suffering from malignant neoplastic disease.

Optionally, a scoring may be done of the detected antigen-antibody complexes according to a standard scoring system known in the art or described herein.

A further aspect is a method for *in vitro* diagnosing and/or prognosing malignant neoplastic disease in a biological sample, the method comprising the steps of
a) contacting said biological sample with a composition as defined herein; or
b) contacting said biological sample with a first targeting agent recognizing a cytokeratin 7 peptide and/or fragment(s) thereof; and
c) contacting said biological sample with a second targeting agent recognizing a cytokeratin 19 peptide and/or fragment(s) thereof: and
d) detecting Heterotypic complex of cytokeratin 7 with cytokeratin 19; wherein steps b) and c) may be performed in any order or, simultaneously; and
e) comparing the amount of heterotypic complex of cytokeratin 7 with cytokeratin19, and/or fragment(s) thereof detected to a positive and/or negative control, thereby diagnosing and/or prognosing the malignant neoplastic disease.

Optionally, a scoring may be done of the detected antigen-antibody complexes according to a standard scoring system known in the art or described herein.
The sample may be any sample possibly comprising malignant neoplastic disease. Further embodiments are wherein the positive control comprises cells from a subject who is suffering from the malignant neoplastic disease. Even further embodiments are wherein the negative control comprises cells from healthy subjects who is not suffering from malignant neoplastic disease.

Thus, the method of diagnosis or prognosis of malignant neoplastic disease by detecting expression or not of heterotypic complex of keratin 7 with keratin 19, and/or fragment(s) thereof within a biological sample of a given subject may be wherein the presence or absence of heterotypic complex of keratin 7 with keratin 19, and/or fragment(s) thereof allows for the diagnosis or prognosis of a subject as healthy or having malignant neoplastic disease. In one embodiment, the methods detect the presence of heterotypic complex of keratin 7 with keratin 19, and/or fragment(s) thereof in a sample wherein the marker is not expressed in healthy, disease-free individuals. In related embodiments, the methods detect elevated levels of heterotypic complex of keratin 7 with keratin 19, and/or fragment(s) thereof that are present at higher levels in samples from individuals that have malignant neoplastic disease such as e.g. lung cancer, bladder cancer, esophagus cancer, hepatocellular cancer, pancreatic cancer, gastric cancer and ovarian cancer as compared to normal, healthy individuals. This is further visualized in the Examples disclosed herein.

In one embodiment, the method of diagnosis or prognosis of malignant neoplastic disease comprises: obtaining a biological sample from a given subject, contacting said sample with the composition disclosed herein under specific binding conditions, allowing the antibodies binding to keratin 7 peptide and/or fragment(s) thereof and keratin 19 peptide and/or fragment(s) thereof to bind to heterotypic complex of keratin 7 with keratin 19, and/or fragment(s) thereof, detecting the antibodies using a detection method, wherein the detection method generates a profile of the expression of said heterotypic complex of keratin 7 with keratin 19, and/or fragment(s) thereof within the sample, transforming the profile generated into a computer-readable form, and comparing the profile of said sample with a database containing profiles from comparable samples specific for healthy subjects, subjects having malignant neoplastic disease. The outcome of said comparison will allow for the determination of whether the subject from which the biological sample was obtained, is healthy or has a malignant neoplastic disease based on the presence, absence or comparative quantity of heterotypic complex of keratin 7 with keratin 19, and/or fragment(s) thereof.

In further embodiments, the detection of heterotypic complex of keratin 7 with keratin 19, and/or fragment(s) thereof may be used in combination with another diagnostic tool to diagnose a subject as being healthy or having malignant neoplastic disease. For example, the detection of heterotypic complex of keratin 7 with keratin 19, and/or fragment(s) thereof may be used in combination with other diagnostic tools specific for lung cancer, bladder cancer, esophagus cancer, and ovarian cancer detection such as, but not limited to, biopsy evaluation, radiography and symptomological evaluation by a qualified clinician.

Physicians routinely use chest radiograph and computed tomography to diagnose patients for lung cancer. Bladder cancer is diagnosed using cystoscopy and urine bound markers like NMP 22 may also aid in the diagnosis, Esophagus cancer is usually diagnosed by esophagogastroduodenoscopy (EGD),or possibly a barium swallow. Ovarian cancer is diagnosed using pelvic examination, ultrasound and CA125. The diagnosis must be confirmed with surgery.

If any of the tests are abnormal the doctors will order a biopsy to confirm their findings. The biopsy tissues are then examined by a pathologist Additional testing may also be used, such as CT-scan or combined CT and PET scans or magnetic resonance imaging (MRI) and other methods known in the clinical practice of mentioned cancers.

The composition comprising antibodies binding specifically to heterotypic complex of keratin 7 with keratin 19, and/or fragment(s) thereof is useful for detecting ambiguous lesions since antibodies binding to heterotypic complex of keratin 7 with keratin 19, and/or fragment(s) thereof will detect only subjects having malignant neoplastic disease and will score negative in ambiguous cases.

The amount of heterotypic complex of keratin 7 with keratin 19, and/or fragment(s) thereof in a sample may be determined using methods well known in the art. Suitable methods for assaying said protein (or antigen) levels in a biological sample include antibody-based techniques. For example, protein expression of the said proteins in tissues can be studied with classical immunochemical and/or immunohistological methods. In these, the specific recognition is provided by the primary antibody (polyclonal or monoclonal) in the composition. A secondary detection system can utilize fluorescent, enzyme, or other conjugated secondary antibodies, as discussed herein.

In one embodiment, the biological samples to be tested are identified as samples associated with malignant neoplastic disease by the up- or down-regulation of heterotypic complex of keratin 7 with keratin 19, and/or fragment(s) thereof level compared to corresponding normal healthy cells. By "up regulated" we mean that heterotypic complex of keratin 7 with keratin 19, and/or fragment(s) thereof is increased by at least 10% compared to expression of the protein in normal (healthy) cells. Similarly, by "down regulated" we mean that the amount of heterotypic complex of keratin 7 with keratin 19, and/or fragment(s) thereof is decreased by at least 10% compared to the expression of heterotypic complex of keratin 7 with keratin 19, and/or fragment(s) thereof in normal (healthy) cells. For example, the level of heterotypic complex of keratin 7 with keratin 19, and/or fragment(s) thereof may be increased by at least 20%, 30%, 40%, 50%, or even 100% or more. Means to measure levels of antigens in samples are enclosed herein and further known in the art.

Detecting the compound or antibody can be achieved using methods well known in the art of clinical imaging and diagnostics further described herein and in the art. The specific method required will depend on the type of detectable label attached to the antibodies of the composition according to the disclosure.

A further aspect is an *in vitro* method for predicting outcome of treatment in a subject of malignant neoplastic disease patients, the method comprising the steps of
a) detecting the expression of heterotypic complex of keratin 7 with keratin 19, and/or fragment(s) thereof in a biological sample obtained from the subject,
b) comparing the expression of heterotypic complex of keratin 7 with keratin 19, and/or fragment(s) thereof to a positive and/or negative control, and thereby predicting the outcome of treatment of the malignant neoplastic disease in said subject based on the detected expression of heterotypic complex of keratin 7 with keratin 19, and/or fragment(s) thereof.

Optionally, a scoring may be done of the detected heterotypic complex of keratin 7 with keratin 19, and/or fragment(s) thereof according to a standard scoring system known in the art or described herein.

The biological sample may be any sample possibly comprising malignant neoplastic disease, preferably a biological sample from a subject having malignant neoplastic disease.

A further aspect is an *in vitro* method of assessing efficacy of treatment of malignant neoplastic disease, the method comprising the steps of
a) providing a biological sample from a subject having malignant neoplastic disease,
b) detecting heterotypic complex of keratin 7 with keratin 19, and/or fragment(s) thereof
c) repeating steps a) and b) at one or more time points during treatment of said subject for malignant neoplastic disease, and wherein a change in relative expression of heterotypic complex of keratin 7 with keratin 19, and/or fragment(s) thereof over time indicates effective treatment.

Thus, an indication of effective treatment is a relative change in decreasing expression of heterotypic complex of keratin 7 with keratin 19, and/or fragment(s) thereof relative an in time previous sample analyzed in the steps of repeating the method.

Optionally, a scoring may be done of the detected antigen-antibody complexes according to a standard scoring system known in the art or described herein.

The sample may be any sample possibly comprising malignant neoplastic disease, preferably a biological sample from a subject having malignant neoplastic disease, and that subject will be, is in-between or is currently under treatment.

A further aspect is an *in vitro* method of assessing recurrence of malignant neoplastic disease, the method comprising the steps of a) providing a biological sample from a subject having previously had malignant neoplastic disease,
b) detecting heterotypic complex of keratin 7 with keratin 19, and/or fragment(s) thereof,
c) repeating steps a) and b) at one or more time points during treatment of said subject for malignant neoplastic disease, and wherein a change in relative expression of heterotypic complex of keratin 7 with keratin 19, and/or fragment(s) thereof over time indicates recurrence of malignant neoplastic disease.

Thus, an indication of recurrence is a relative change in increasing amounts of heterotypic complex of keratin 7 with keratin 19, and/or fragment(s) thereof that identify malignant neoplastic disease, i.e. an over-time increase in expression of heterotypic complex of keratin 7 with keratin 19, and/or fragment(s) thereof relative an in time previous sample analyzed in the steps of repeating the method.

The methods provided herein may be performed manually, or, preferably, on an automated reading device. Thus, in one embodiment the methods are performed manually. In further embodiments, the methods are performed on an automated reading device.

### Uses of the composition

Further aspects of the present disclosure include uses of the methods and composition provided herein.

A further aspect of the present disclosure is use of the methods provided herein to detect heterotypic complex of keratin 7 with keratin 19, and/or fragment(s) thereof. A further aspect of the present disclosure is use of the methods provided herein to detect malignant neoplastic disease such as e.g. lung cancer, bladder cancer, esophagus cancer or ovarian cancer.

A further aspect of the present disclosure is use of the methods to diagnose or prognose malignant neoplastic disease such as e.g. lung cancer, bladder cancer, esophagus cancer or ovarian cancer.

A further aspect of the present disclosure is use of the methods to predict outcome of treatment of malignant neoplastic disease such as e.g. lung cancer, bladder cancer, esophagus cancer or ovarian cancer.

A further aspect of the present disclosure is use of the methods to assess efficacy of treatment of malignant neoplastic disease such as e.g. lung cancer, bladder cancer, esophagus cancer or ovarian cancer.

A further aspect of the present disclosure is use of the methods to assess recurrence of malignant neoplastic disease such as e.g. lung cancer, bladder cancer, esophagus cancer or ovarian cancer.

### Kits

The present disclosure also provides kits for immunoassays such as immunohistochemistry. Thus, a further aspect of the present disclosure provides a kit for immunoassays comprising
a) the composition of the present disclosure provided herein; or
b) a first container comprising a first targeting agent recognizing a keratin 7 peptide and/or fragment(s) thereof as defined herein; and
c) a second container comprising a second targeting agent recognizing a keratin 19 peptide and/or fragment thereof as defined herein, and
d) optionally instructions for performing methods as defined herein.

Further embodiments include visualization reagents to be able to detect the composition binding specifically to heterotypic complex of keratin 7 with keratin 19, and/or fragment(s) thereof. Examples of visualization and detection reagents are known in the art.

In some kit embodiments, the primary antibody or antibodies can be directly labelled as described herein.

Other kit embodiments will include secondary or further detection such as secondary antibodies (e.g., goat anti-rabbit antibodies, rabbit anti-mouse antibodies, anti-hapten antibodies) or non-antibody hapten-binding molecules (e.g., avidin or streptavidin) as described herein. In such kits, the secondary or further detection means may be directly labelled with a detectable moiety. In other instances, the secondary (or further) antibody or binding agent will be conjugated to a hapten (such as biotin, DNP, and/or FITC), which is detectable by a detectably labelled cognate hapten binding molecule (e.g., streptavidin (SA) horseradish peroxidase, SA alkaline phosphatase). Some kit embodiments may include colorimetric reagents (e.g., DAB, and/or AEC) in suitable containers to be used in concert with primary or secondary (or higher order) detection means (e.g., antibodies or binding entities) that are labelled with enzymes for the development of such colorimetric reagents.

In some embodiments, a kit includes positive or negative control samples, such as a cell line, tissue or body fluid known to express or not express heterotypic complex of keratin 7 with keratin 19, and/or fragment(s) thereof. Examples of control samples include but are not limited to normal (e.g., non-cancerous) cells or tissues, malignant neoplastic samples from subject known not to have or have had any recurrence of malignant neoplastic disease following treatment (e.g., at least 5 years or at least 10 years following treatment).

In some embodiments, a kit includes instructional materials disclosing, for example, means of use of the composition or further binding entities or detection means, e.g. an antibody that specifically binds heterotypic complex of keratin 7 with keratin 19, and/or fragment(s) thereof or means of use for a particular reagent. The instructional materials may be written, in an electronic form (e.g., computer diskette or compact disk) or may be visual (e.g., video files). The kits may also include additional components to facilitate the particular application for which the kit is designed. Thus, for example, the kit can include buffers and other reagents routinely used for the practice of a particular disclosed method. Such kits and appropriate contents are well known to those of skill in the art. The kit may further comprise, in an amount sufficient for at least one assay, the composition according to the disclosure as a separately packaged reagent.

Instructions for use of the packaged reagent are also typically included. Such instructions typically include a tangible expression describing reagent concentrations and/or at least one assay method parameter such as the relative amounts of reagent and sample to be mixed, maintenance time periods for reagent/sample admixtures, temperature, buffer conditions and the like.

A further aspect of the present disclosure provides a kit for detection of heterotypic complex of keratin 7 with keratin 19, and/or fragment(s) thereof in a biological sample *in vitro*, the kit comprising
a) the composition of the present disclosure provided herein; or
b) a first container comprising a first targeting agent recognizing a keratin 7 peptide and/or fragment(s) thereof as defined herein; and
c) a second container comprising a second targeting agent recognizing a keratin 19 peptide and/or fragment thereof as defined herein, and
d) optionally instructions for performing methods as defined herein..

A further aspect of the present disclosure provides a kit for detection of malignant neoplastic disease such as e.g. lung cancer, bladder cancer, esophagus cancer or ovarian cancer in a biological sample *in vitro,* the kit comprising
a) the composition of the present disclosure provided herein; or
b) a first container comprising a first targeting agent recognizing a keratin 7 peptide and/or fragment(s) thereof as defined herein; and
c) a second container comprising a second targeting agent recognizing a keratin 19 peptide and/or fragment thereof as defined herein, and
d) optionally instructions for performing methods as defined herein.

A further aspect of the present disclosure provides a kit for diagnosing and/or prognosing malignant neoplastic disease such as e.g. lung cancer, bladder cancer, esophagus cancer or ovarian cancer in a biological sample *in vitro,* the kit comprising
a) the composition of the present invention provided herein; or
b) a first container comprising a first targeting agent recognizing a keratin 7 peptide and/or fragment(s) thereof as defined herein; and
c) a second container comprising a second targeting agent recognizing a keratin 19 peptide and/or fragment thereof as defined herein, and
d) optionally instructions for performing methods as defined herein.

A further aspect of the present disclosure provides a kit for predicting outcome of treatment in a subject of malignant neoplastic disease such as e.g. lung cancer, bladder cancer, esophagus cancer or ovarian cancer patients, the kit comprising
a) the composition of the present disclosure provided herein; or
b) a first container comprising a first targeting agent recognizing a keratin 7 peptide and/or fragment(s) thereof as defined herein; and
c) a second container comprising a second targeting agent recognizing a keratin 19 peptide and/or fragment thereof as defined herein, and
d) optionally instructions for performing methods as defined herein.

A further aspect of the present disclosure provides a kit for assessing efficacy of treatment of malignant neoplastic disease such as e.g. lung cancer, bladder cancer, esophagus cancer or ovarian cancer, the kit comprising
a) the composition of the present disclosure provided herein; or
b) a first container comprising a first targeting agent recognizing a keratin 7 peptide and/or fragment(s) thereof as defined herein; and
c) a second container comprising a second targeting agent recognizing a keratin 19 peptide and/or fragment thereof as defined herein, and
d) optionally instructions for performing methods as defined herein.

A further aspect of the present disclosure provides a kit for assessing recurrence of malignant neoplastic disease such as e.g. lung cancer, bladder cancer, esophagus cancer or ovarian cancer, the kit comprising
a) the composition of the present disclosure provided herein; or
b) a first container comprising a first targeting agent recognizing a keratin 7 peptide and/or fragment(s) thereof as defined herein; and
c) a second container comprising a second targeting agent recognizing a keratin 19 peptide and/or fragment thereof as defined herein, and
d) optionally instructions for performing methods as defined herein.

Certain kit embodiments can include a carrier means, such as a box, a bag, a satchel, plastic carton (such as moulded plastic or other clear packaging), wrapper (such as, a sealed or sealable plastic, paper, or metallic wrapper), or other container.

In some examples, kit components will be enclosed in a single packaging unit, such as a box or other container, which packaging unit may have compartments into which one or more components of the kit can be placed. In other examples, a kit includes a one or more containers, for instance vials, tubes, and the like that can retain, for example, one or more biological samples to be tested.

The kit embodiments include, for instance, syringes, cotton swabs, or latex gloves, which may be useful for handling, collecting and/or processing a biological sample. Kits may also optionally contain implements useful for moving a biological sample from one location to another, including, for example, droppers, syringes, and the like. Still other kit embodiments may include disposal means for discarding used or no longer needed items (such as subject samples, etc.). Such disposal means can include, without limitation, containers that are capable of containing leakage from discarded materials, such as plastic, metal or other impermeable bags, boxes or containers.

Non-limiting examples which embody certain aspects of the invention will now be described.

### EXAMPLE 1

### Assay for determination of heterotypic complex of keratin 7 with keratin 19 in body fluids

The purpose was to create an assay suitable for clinical evaluation.

Monclonal antibody Ks 7.18 specific for keratin 7 was conjugated to biotin and monoclonal antibody BM 19.21 specific to keratin 19 was conjugated to peroxidase according to methods described in "Recent development in the periodate method of conjugating horseradish peroxidase (HRP) to antibodies" (M Barbara Wilson and Paul K. Nakane, Elsevier/North-Holland Biomedical Press, 1978).

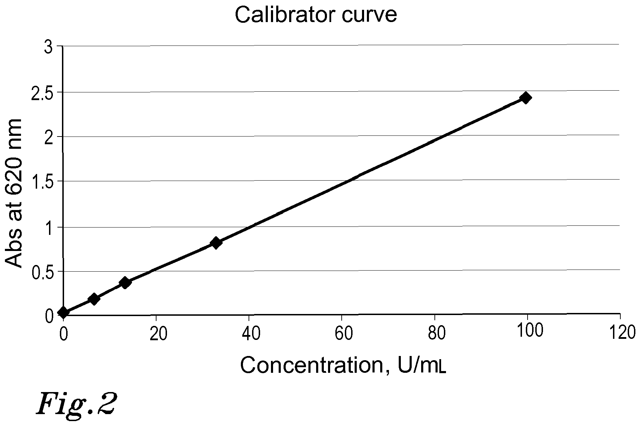

A sandwich enzyme-immunoassay was carried out to determine the presence of heterotypic complex of keratin 7 with keratin 19 or fragments thereof in body fluids obtained from patients diagnosed with malignant neoplastic disease, or obtained from patients diagnosed with non-malignant disease or from individuals considered healthy. In this procedure 100 µl of antibody solution in a BSA Tris buffer pH 7.2 containing the two monoclonal antibodies, biotin conjugated Ks 7.18 (1µg/ml) and peroxidase conjugated BM 19.21 (2 µg/ml), was incubated together with 50 µl serum sample or calibrator, i.e. samples with known concentrations of heterotypic complex of keratin 7 with keratin 19 or fragments thereof, for one hour shaking at room temperature in streptavidin-coated wells of a micro-plate. After incubation the wells were washed six times with 0.05% Tween/PBS to remove excess antibody. Subsequently 100 µl buffered TMB substrate (hydrogen peroxide and 3, 3', 5, 5' tetramethylbenzidine) was added to the wells for the enzyme reaction to proceed at room temperature for 30 min. During the enzyme reaction blue color developed in well if antigen, i.e. heterotypic complex of keratin 7 with keratin 19 or fragments thereof is present in the serum sample. The color intensity was determined in a microplate reader at 620 nm. Calibration curves were constructed for each assay by plotting absorbance value versus the concentration for each calibrator. The concentrations of heterotypic complex of keratin 7 with keratin 19 or fragments thereof present in patient samples were determined from the calibration curve. The assay as described in this example is hereinafter called the "K7/K19 assay". Figure 2 shows a typical calibrator curve.

### EXAMPLE 2

### Sensitivity of the K7/K19 assay for different forms of cancers

In total 398 sera from patients suffering from various forms of cancer were tested using the K7/19 assay described in Example 1. The results are summarised in table 1.

In conclusion the K7/K19 assay showed the highest clinical sensitivity for bladder cancer, ovarian cancer, lung cancer and oesophagus cancer compared to the other cancers studied, and especially samples from lung cancer, bladder cancer and ovarian cancer patients had high concentration of heterotypic complex of keratin 7 with keratin 19 (or fragment(s) thereof). In contrast the K7/K19 assay showed sensitivities as low as 10% or less for Squamous Cell Carcinoma of the Cervix, and Breast, as well as Head & neck cancer and cancers of the gastrointestinal tract. Concentration values of heterotypic complex of keratin 7 with keratin 19 (or fragment(s) thereof) for these latter cancer patients were in the same range as those of the controls groups (healthy and benign) in table 2.The K7/K19 assay is thus not generally suited for these types of cancers because of the low sensitivities. However, two breast cancer patients and one GI tract cancer patient had values higher than the highest values from the samples in the benign disease group. These high values may indicate an aggressive cancer or poor prognosis. That is to say that although the assay is not generally suited for some cancers, the few cases where the assay indeed gives high values may be of clinical value.

**Table 1: Sensitivity versus 96% specificity for various cancers.**

| **Cancer group** | **No. of Samples** | **Upper 95th percentile (U/mL)** | **Mean (U/mL)** | **No of positive at cut-off of 1.1 U/mL** | **Positive tests (%)** |
|---|---|---|---|---|---|
| Lung | 119 | 38,1 | 8,0 | 43 | **36,1** |
| Ovarian | 40 | 13,3 | 2,38 | 15 | **37,5** |
| Esophagus (SCC) | 40 | 4,3 | 1,30 | 18 | **45** |
| Bladder | 38 | 47,0 | 8,16 | 19 | **50** |
| GI Tract | 40 | 1,9 | 0,39 | 3 | **7,5** |
| Cervix (SCC) | 40 | 1,8 | 0,35 | 4 | **10** |
| Breast | 40 | 4,9 | 0,65 | 3 | **7,5** |
| Head, neck | 40 | 1,2 | 0,07 | 2 | **5** |

### EXAMPLE 3:

### Establishment of a reference range for healthy individuals.

One hundred and twelve (112) sera from apparently healthy individuals were tested according to the K7/K19 assay as described in Example 1 to establish a reference range for individuals having no apparent signs of malignant neoplastic disease. 95% of the individuals had a concentration of heterotypic complex of keratin 7 with keratin 19 or fragment(s) thereof that was less than 1.01 U/mL, or 95.5 % had a value of 1.09 U/mL or less. Five (5) of the 112 healthy individuals (i.e. 4.5%) had a concentration above 1.1 U/mL. The mean concentration of heterotypic complex of keratin 7 with keratin 19 or fragment(s) thereof for the tested individuals was 0.15 U/mL (see table 2).

### EXAMPLE 4:

### Establishment of reference ranges for individuals with benign (non-cancerous) neoplastic disease.

In total 239 sera from patients suffering from diseases, known to generally give elevated tumour marker values, were tested in the K7/19 assay according to example 1. The results are shown in the table 2 below.

In conclusion the 95^{th} percentile value was not higher for the benign diseases than for the healthy blood donors. All groups gave 5% or less positive samples using a cut value of 1.09 U/mL and 99% of the samples from the benign group had values below 1.8 U/mL (not shown).

**Table 2: Reference values and % positive samples for healthy blood donors and benign diseases respectively**

| **Group** | **No of samples** | **Upper 95th percentile (U/mL)** | **Mean (U/mL)** | **No of positive at cut-off of 1.1 U/mL** | **Positive tests (%)** |
|---|---|---|---|---|---|
| **Healthy blood donors** | 112 | **1,01** | 0,15 | 5 | **4,5** |
| **Benign disease** | | | | | |
| Lung | 75 | 1,08 | 0,36 | 2 | **2,7** |
| Gynecological | 45 | 1,40 | 0,16 | 2 | **4,4** |
| Liver | 39 | 1,44 | 0,19 | 2 | **5,1** |
| Kidney | 40 | 1,52 | 0,55 | 2 | **5** |
| CHF | 40 | 0,85 | 0,25 | 1 | **2,5** |
| **All Benign** | 239 | **1,03** | 0,31 | 9 | **3,8** |

### EXAMPLE 5A:

### Test of possible cross-reactivity with heterotypic complex of keratin 8 with keratin 19 or keratin 19.

A calibrator containing heterotypic complex of keratin 8 with keratin19, i.e. CYFRA 21-1 kit calibrator (described in: Lung cancer-associated keratin 19 fragments: development and biochemical characterisation of the new serum assay Enzymun-Test CYFRA 21-1. Bodenmuller H, Ofenloch-Hahnle B, Lane EB, Dessauer A, Böttger V, Donié F, Int J Biol Markers. 1994 Apr-Jun; 9(2):75-81) having a keratin 19 concentration of 50 µg/L as determined by the CYFRA 21-1 EIA kit (Fujirebio Diagnostics Inc.) was assayed, as described in Example 1, together with a blood donor sample, calibrator 0 in the K7/K19 assay (which consists of calibrator matrix free of keratin 7/19) and a positive control consisting of sera from an ovarian cancer patient.

**Table 3: results for samples in the K7/19 assay**

| **Sample** | **A620** |
|---|---|
| Calibrator 0 | 0,040 |
| CYFRA 21-1 Calibrator 50 µg/L | 0.048 |
| Blood donor | 0.060 |
| Ovarian cancer | 0.267 |

Conclusion: No significant signal was detected in the assay, or the concentration detected was low enough for the CYFRA 21-1 Calibrator 50 µg/L, that any cross-reactivity to K19 or heterotypic complex of keratin 8 with keratin 19 would influence clinical performance. This is evident since the highest calibrator from the CYFRA kit (50µg/L) was around the detection limit for the K7/19 assay and at the same time also lower than the blood donor sera and much lower than the positive control consisting of the ovarian cancer sera.

### EXAMPLE 5B

### Comparison between Ks 19.2 and BM 19.21 clone

An assay was constructed and run as in example 1, but Keratin 19 monoclonal antibody clone Ks 19.2 was used instead of clone BM 19.21. This assay was compared to the assay having Keratin 19 monoclonal antibody clone BM 19.21. The assays were run on the same samples. Fifteen (15) sera from individuals suffering from cancer (lung cancer, esophagus cancer, bladder cancer or ovarian cancer) and 13 sera patients suffering from benign disease (liver-, kidney-, lung-, or CHF) were tested according to the K7/K19 assay as described in Example 1.

### Results:

There was a high correlation (r=0.97) between the assays and number of positive samples were:

| | Positive cancer | Positive benign |
|---|---|---|
| Ks 7.18/BM 19.21 | 14/15 | 2/13 |
| Ks 7.18/Ks 19.2 | 12/15 | 0/13 |

Conclusion: There is no significant difference between the two assays. Any of the "BM 19.21like" monoclonal antibodies may be used to demonstrate the invention.

### EXAMPLE 6

### Comparison with the established Keratin 19 marker CYFRA 21-1

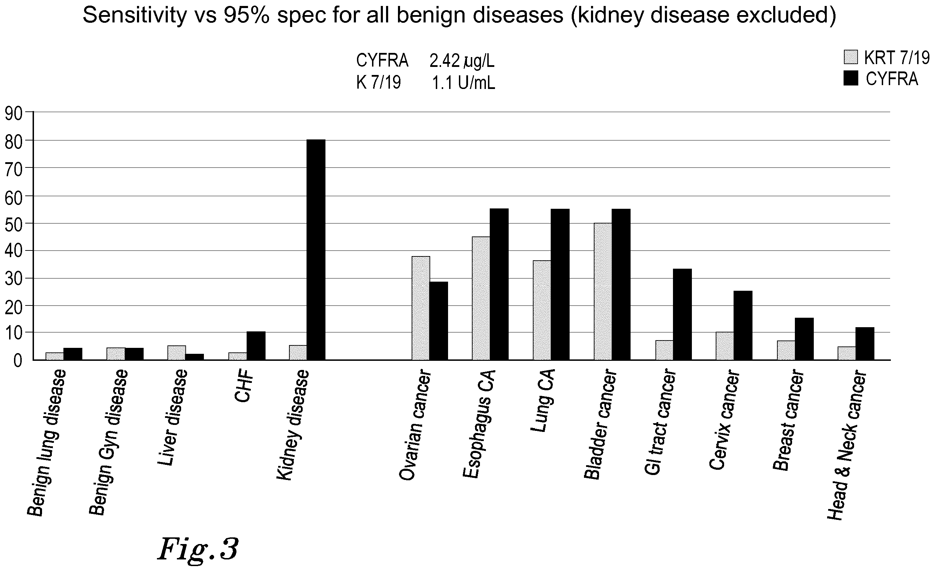

The same samples assayed in examples 3 and 4 were tested with the CYFRA 21-1 Enzyme immunometric assay (EIA) according to the instructions for use as included (Fujirebio Diagnostics Inc.). The CYFRA 21-1 EIA provides a quantitative determination of soluble keratin 19 fragments in human serum, and is frequently used as an aid in monitoring disease progression during the course of disease and treatment in lung cancer patients. An upper 95^{th} percentile reference limit of 2.42 µg/L was calculated for the CYFRA 21-1 EIA based on the benign diseases, with kidney disease excluded (n=199). The results are shown graphically in figure 3. Figure 3 shows Sensitivity vs 95% specificity for benign diseases (kidney disease excluded). The cut-off values are 2.42 µg/L for the CYFRA 21-1 EIA and 1.1 U/mL for the K7/19 assay.

Conclusion: The K7/19 assay shows a superior specificity compared to the CYFRA 21-1 EIA. From figure 3 it is seen that levels of the tumour marker CYFRA 12-1 (keratin 19) is elevated in patients suffering from Kidney disease, but also in patients with cardiac heart failure. The K7/19 assay is also more tissue restricted, but having comparable sensitivities in ovarian cancer, oesophagus cancer (SCC) and urinary bladder cancer. The sensitivity in lung cancer, all histotypes, is lower than in CYFRA 21-1.

### EXAMPLE 7

### Evaluation of the K7/K19 assay for ovarian cancer and a comparison with established markers.

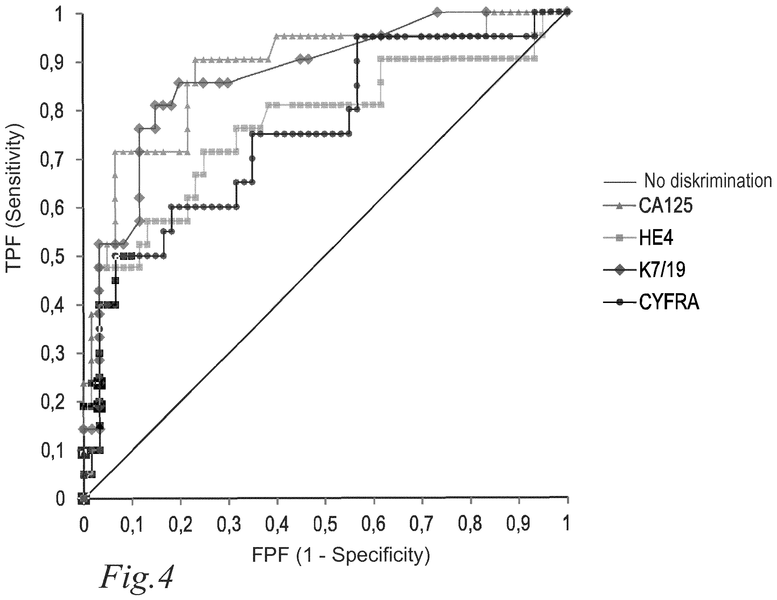

Serum samples from women visiting the hospital for an abnormal growth in the lower abdomen or pelvic region (pelvic mass) were run in the following assays: CA 125 EIA, HE4 EIA, CYFRA 21-1 EIA (all of which are commercially available from Fujirebio Diagnostics Inc.) and the K7/19 assay according to example 1. The CA 125 EIA is specific for the CA125 antigen and HE4 EIA is specific for the HE4 antigen. Both tests are frequently used to monitor patients with gynaecological malignancies such as epithelial ovarian cancer. The samples, being well characterized, are described in table 3 below. It turned out that in this sample set 21 patients had ovarian cancer and 60 patients had benign disease. A Receiver operating characteristic (ROC) Curve was created comparing the sensitivity vs. specificity profiles for the four assays: CA 125 EIA, HE4 EIA, CYFRA 21-1 EIA in ovarian cancer as seen in figure 4.

The CA125 EIA and K7/19 assay gave the highest sensitivity, 52%, at 95% specificity and the greatest area under the curve, thus demonstrating the higher ability to discriminate between ovarian cancer and benign pelvic masses compared to the HE4 EIA or CYFRA 21-1 EIA.

**Table 4 Patient material for ovarian cancer study**

| **Diagnosis** | **Pathology** | **No. of samples** |
|---|---|---|
| Epithelial Ovarian cancer | serous | 14 |
| Epithelial Ovarian cancer | endometrioid | 2 |
| Cancer - Non-Epithelial Ovarian | granulosa, immature teratoma | 2 |
| Epithelial Ovarian cancer | mucinous | 3 |
| | | **21** |
| Benign | adenofibroma | 2 |
| Benign | simple cyst | 6 |
| Benign | cystadenoma | 7 |
| Benign | dermoid | 3 |
| Benign | endometriosis | 2 |
| Benign | endometrioma | 3 |
| Benign | fibroma | 2 |
| Benign | hydrosalpinx | 2 |
| Benign | leiomyomata | **1** |
| Benign | mature cystic teratoma | 2 |
| Benign | mucinous cystadenoma | 3 |
| Benign | myoma | 2 |
| Benign | normal ovary with hemorrhagic follicle | **1** |
| Benign | ovarian cyst | **1** |
| Benign | paratubal cyst | **1** |
| Benign | myoma | 2 |
| Benign | serous cyst | 4 |
| Benign | serous cystadenofibroma | 2 |
| Benign | serous cystadenoma | 7 |
| Benign | teratoma | 2 |
| Benign | not specified | 5 |
| **60** | | |

### EXAMPLE 8

### Combination of biomarkers from example 7.

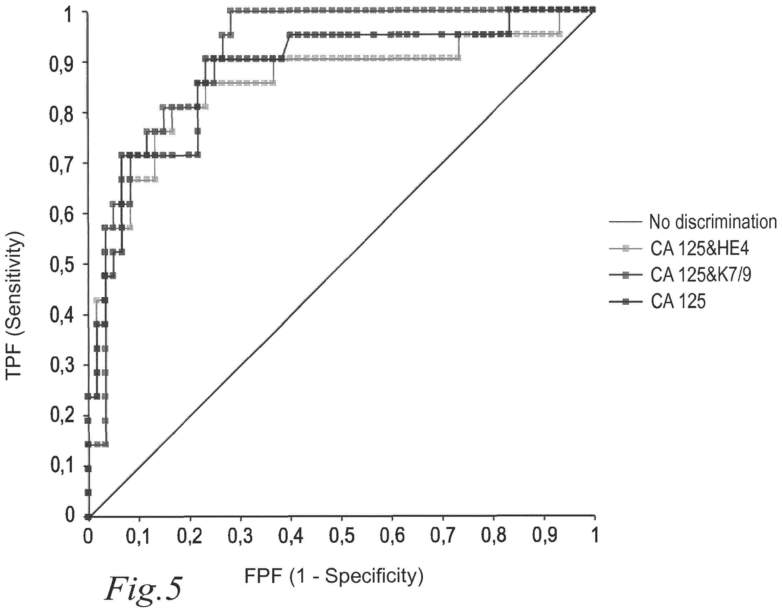

Figure 5 shows a ROC-curve comparing the combination CA125 EIA & HE4 EIA with the combination CA125 EIA & K7/19 assay. If the CA125 EIA was combined with the K7/19 assay the diagnostic sensitivity is increased compared to when the CA 125 EIA or K7/19 assay are used alone.

The sensitivity for the *CA125 EIA* & *K7*/*19 assay* combination is 62% at 95% specificity and a sensitivity of 100% is reached at a specificity of 70%.

Conclusion: examples 7 and 8 demonstrate that the K7/19 assay may be of clinical use in management of ovarian cancer patients. Due to the relatively high cancer specificity of the K7/19 assay the use of heterotypic complex of keratin 7 with keratin 19 as a marker for malignant neoplastic disease such as e.g. lung cancer, bladder cancer, esophagus cancer, and ovarian cancer can increase the sensitivity without decreasing the specificity.

### EXAMPLE 9

### Lung cancer: differential diagnosis between NSCLC and SCLC

Twenty nine (29) sera from Small Cell Lung Cancer patients were tested according to the K7/K19 assay as described in Example 1. Twenty eight samples had concentrations of heterotypic complex of keratin 7 with keratin 19 (or fragment(s) thereof) that were below 1.1 U/mL. i.e. only one of samples was positive in the K7/19 assay. The samples were also previously analysed for ProGRP and 17 out of the 29 samples had ProGRP concentrations above 200 pg/mL demonstrating that samples were taken from individuals with active SCLC.

The K7/K19 assay is able to distinguish between Non-small cell lung cancer (NSCLC) and Small Cell Lung cancer (SCLC). This in combination with the high specificity of the K7/19 assay makes the K7/K19 assay suitable as a tool to aid in the differential diagnosis between Small Cell and Non-Small Cell Lung Cancer. The K7/K19 assay will be especially useful in combination with ProGRP, an assay with high sensitivity and specificity for Small Cell Lung cancer (Tang Jian-hua, et. al. Meta analysis, Diagnostic value of tumours marker pro-gastrin-releasing peptide in patients with small cell lung cancer: a systematic review, Chinese Medical Journal 2011; 124(10): 1563-1568.). CYFRA 21-1 detects also SCLC and is therefore not useful for such an application.

It can also be anticipated that combined measurement of ProGRP and K7/19 can be of value to differentiate between SCLC and combined SCLC (c-SCLC) and between Large cell Neuroendocrine carcinoma (LC-NET) and SCLC.

### EXAMPLE 10

### Diagnostic potential of the K7/K19 assay in Lung cancer.

The data from Example 3 (112 samples from healthy individuals), Example 4 (239 samples of benign diseases), and Example 5 (398 samples of malignant neoplastic diseases) were exploited using a cut-off value four times the cut-off value used for the benign diseases (kidney diseases were excluded in the CYFRA 21-1 EIA to not discredit this assay), giving cut-off values: 4.4 U/mL for the K7/19 assay and 9.7 µg/L for the CYFRA 21-1 EIA. With these limits the number of positive lung cancer samples, other positive cancer samples (i.e. not lung cancer samples) and positive samples from patients suffering from benign diseases are summarized in table 5.

**Table 5 positive samples in the K7/19 assay and CYFRA 21-1 EIA using diagnostic cut off values**

| **Assay** | No of positive lung cancer samples out of a total of 119 | No of positive other cancer samples out of a total of 280 | No of positive benign diseases out of a total of 240 | PPV Lung cancer |
|---|---|---|---|---|
| K7/19 | 26 (22%) | 22 (8%) | 0 | 54% |
| CYFRA 21-1 | 23 (19%) | 28 (10%) | 1 | 45% |

| | | | | |
|---|---|---|---|---|
| PPV Lung cancer = positive predictive value for lung cancer (i.e. positive lung cancer samples/ all positive samples) | | | | |

Conclusion: Although the percentage of samples that are positive is quite low, samples being positive are with high probability cancer. In the case of K7/K19 assay there is more than a 50% probability that a sample that has a value for heterotypic complex of keratin 7 with keratin 19 (or fragment(s) thereof) that is above 4.4 U/mL, is lung cancer. For the CYFRA 21-1 EIA this probability is lower and also the possibility of kidney failure cannot be ruled out (the positive benign sample). In this preliminary data set the histological types of lung cancer were not known.

### EXAMPLE 11

### Potential of the K7/K19 assay in biliary tract cancers

Sera from patients with advanced confirmed epithelial cancer in the biliary tract or confirmed advanced gastric cancer were run in the K7/19 assay as described in Example 1.

| **Malignant disease** | **Number of sera evaluated** | **Positive sera in K7/K19** |
|---|---|---|
| Pancreatic carcinoma | 4 | 3 |
| Gall bladder cancer | 2 | 2 |
| Primary liver cell cancer | 1 | 1 |
| Stomach cancer | 4 | 3 |

Conclusion: A K7/K19 assay may be of clinical value also in the management of patients suffering from the above mentioned epithelial cancers.

### EXAMPLE 12 Nytt

### Alternative Assays for determination of heterotypic complex of keratin 7 with keratin 19 in serum.

The purpose was to compare assays suitable for clinical evaluation having major different epitope locations for the antibodies compared to the Ks7.18/BM 19.21 assay described in example 1.

Antibody Ks.19.1, specific for keratin 19, binds to the amino acid sequence 311-335 on keratin 19 (Böttger et al. Eur. J. Biochem. 231, 475-485, 1995) and monoclonal antibody RCK 105 is specific for keratin 7 (Ramaekers et al. Exp Cell Res. 170(1):235-49, 1987).

Monoclonal antibody RCK105 was conjugated to biotin and monoclonal antibody BM Ks 19.1 was conjugated to peroxidase according to methods described in "Recent development in the periodate method of conjugating horseradish peroxidase (HRP) to antibodies" (M Barbara Wilson and Paul K. Nakane, Elsevier/North-Holland Biomedical Press, 1978).

A sandwich enzyme-immunoassay was carried out, as in example 1, to determine the presence of heterotypic complex of keratin 7 with keratin 19 or fragments thereof in serum obtained from patients diagnosed with malignant neoplastic disease. The two assays were compared for the same 19 cancer samples.

There was a high correlation between the assays r=0.98, n=19 samples, and no major clinical discrepancy could be demonstrated as seen in the table below.

The results are summarized in the table below.

| Mab combination (K7 / K19) | Malignant disease | Positive | / examined sera |
|---|---|---|---|
| Ks 7.18 / BM 19.21 | Ovarian cancer | 7 | / 7 |
| Ks 7.18 / BM 19.21 | Bladder cancer | 6 | / 6 |
| Ks 7.18 / BM 19.21 | Lung cancer | 6 | / 6 |
| RCK 105 / Ks 19.1 | Ovarian cancer | 5 | / 7 |
| RCK 105 / Ks 19.1 | Bladder cancer | 6 | / 6 |
| RCK 105 / Ks 19.1 | Lung cancer | 5 | / 6 |

Conclusion: An alternative assay for Keratin 7/19 may be constructed by using a keratin19 antibody that binds to amino acids 311-331 on keratin 19 in pair with RCK 105, having its epitope located to another part of keratin 7 than antibody Ks 7.18.

### EXAMPLE 13

Epitope location for antibody Ks 7.18 on keratin 7 could be established by complete inhibition by the Ks 19.1 keratin 19 antibody in the assay for heterotypic complex of keratin 7 with keratin 19.

An assay was constructed as in example 1, but with Ks 19.1 mab conjugated to peroxidase instead of BM 19.21 mab. The results for the two different keratin 19 antibodies in combination with the Ks 7.18 antibody is shown in the table below for the defined samples.

| Sample | Ks 7.18 / Ks 19.1 (A620nm) | Ks 7.18 / BM 19.21 (A620nm) |
|---|---|---|
| Calibrator 0 | 0.04 | 0.04 |
| Blood donor sera | 0.04 | 0.04 |
| Cancer sera 1 | 0.04 | 0.32 |
| Cancer sera 2 | 0.04 | 0.27 |
| Pleura 1 from lung cancer | 0.06 | 3.57 |
| Pleura 2 from lung cancer | 0.07 | 3.47 |

### Conclusion:

Because the heterotypic complex between keratin 7 and keratin 19 is aligned in parallel and in register with the helical structures any two antibodies binding to corresponding exposed positions, starting from helix 1A to helix 2B, on keratin 7 and keratin 19 respectively will not bind simultaneously. i.e. where the keratin 7 is coiled around keratin 19 those corresponding positions would not allow simultaneous binding for steric reasons. This is further exemplified by that e.g. amino acids 271- 291 on keratin 19 pair up in the coiled coil with amino acids 283-303 on keratin 7.

The epitope for BM 19.21 is located on amino acid sequence 352-368 on keratin 19. The epitope for Ks 19.1 antibody on keratin 19 has been located to amino acid sequence 311-335. The corresponding sequence on Keratin 7 is 323-347.

It can be concluded that the epitope for Ks 7.18 is located on amino acid sequence from 300-350 on Keratin 7.

## Claims

1. A composition comprising at least two antibodies, antigen-binding fragment(s), or combinations thereof, wherein at least one first antibody, antigen-binding fragment, or combination thereof recognizes a sequence located within the 256-412 amino acid sequence, or more preferably within the 300-380 amino acid sequence in the keratin 7 moiety of the heterotypic complex of keratin 7 with keratin 19 and/or fragment(s) thereof, and at least one second antibody, antigen-binding fragment, or combination thereof recognizes a sequence located within the 244-400 amino acid sequence, or more preferably within the 311-375 amino acid sequence in the keratin 19 moiety of the heterotypic complex of keratin 7 with keratin 19 and/or fragment(s) thereof, **characterized in that** said first and second antibodies, antigen-binding fragment(s), or combination thereof are capable of binding specifically and simultaneously to heterotypic complex of keratin 7 with keratin 19 and/or fragment(s) thereof.

2. The composition according to claim 1, wherein the first antibody is selected from the group consisting of the antibodies KS 7.18 and RCK105 or antigen-binding fragment(s) variants, fusions, derivatives or combination thereof and recognizes a sequence of at least 3, or at least 5, or at least 7, or 10 or more amino acids located on the keratin 7 moiety of the heterotypic complex of keratin 7 with keratin 19.

3. The composition according to any one of claims 1-2, wherein the second antibody is selected from the group consisting of the antibodies A53-B/A2aka Ks19.1, BM-19.21, Ks19.2, or antigen-binding fragment(s) variants, fusions, derivatives or combination thereof, and recognizes a sequence of at least 3, or at least 5, or at least 7, or 10 or more amino acids located on the keratin 19 moiety of the heterotypic complex of keratin 7 with keratin 19.

4. The composition according to any one of claims 1-3, wherein the first antibody specifically binds amino acid sequence 300-350 on keratin 7, and the second antibody specifically binds amino acid sequence 346-367 on keratin 19.

5. The composition according to any one of claims 1-4, wherein the first antibody is the Ks 7.18 monoclonal antibody and the second antibody is the BM-19.21 monoclonal antibody.

6. The composition according to any one of claims 1-3, wherein the first antibody is the RCK105 monoclonal antibody and the second antibody is the Ks 19.1 monoclonal antibody.

7. An *in vitro* method for detecting a heterotypic complex of keratin 7 with keratin 19 and/or fragment(s) thereof in a biological sample, said method comprising the steps of:
a) contacting said biological sample with a composition comprising at least two antibodies, antigen-binding fragment(s), or combinations thereof, wherein at least one first antibody, antigen-binding fragment, or combination thereof recognizes a sequence located within the 256-412 amino acid sequence, or more preferably within the 300-380 amino acid sequence in the keratin 7 moiety of the heterotypic complex of keratin 7 with keratin 19 and/or fragment(s) thereof, and at least one second antibody, antigen-binding fragment, or combination thereof recognizes a sequence located within the 244-400 amino acid sequence, or more preferably within the 311-375 amino acid sequence in the keratin 19 moiety of the heterotypic complex of keratin 7 with keratin 19 and/or fragment(s) thereof, said first and second antibodies, antigen-binding fragment(s), or combination thereof-are capable of binding specifically and simultaneously to heterotypic complex of keratin 7 with keratin 19 and/or fragment(s) thereof; or alternatively
b) contacting said biological sample with a first antibody, antigen-binding fragment, or combination thereof recognizes a sequence located within the 256-412 amino acid sequence, or more preferably within the 300-380 amino acid sequence in the keratin 7 moiety of the heterotypic complex of keratin 7 with keratin 19 and/or fragment(s) thereof; and
c) contacting said biological sample with a second antibody, antigen-binding fragment, or combination thereof recognizes a sequence located within the 244-400 amino acid sequence, or more preferably within the 311-375 amino acid sequence in the keratin 19 moiety of the heterotypic complex of keratin 7 with keratin 19 and/or fragment(s) thereof: and
d) detecting said heterotypic complex of keratin 7 with keratin 19 and/or fragment(s) thereof;
wherein steps b) and c) may be performed in any order or, simultaneously.

8. The method according to claim 7, wherein the biological sample is a biological fluid sample of the group consisting of blood serum, blood plasma, lymph, exudates, feces, gastric acid, gastric juice, lymph, mucus, pericardial fluid, peritoneal fluid, pleural fluid, pus, saliva, sputum, synovial fluid, tears, sweat, vaginal secretion, vomit and urine, preferably the biological fluid sample is selected from blood, serum, and/or plasma.

9. The method according to any one of claims 7-8, wherein the first antibody is selected from the group consisting of the antibodies KS 7.18 and RCK105 or antigen-binding fragment(s) variants, fusions, derivatives or combination thereof, and recognize a sequence of at least 3, or at least 5, or at least 7, or 10 or more amino acids located on the keratin 7 moiety of the heterotypic complex of keratin 7 with keratin 19.

10. The method according to any one of claims 7-8, wherein the second antibody is selected from the group consisting of the antibodies A53-B/A2aka Ks19.1, BM-19.21, Ks19.2, or antibody fragment(s) variants, fusions, derivatives or combination thereof, and recognizes a sequence of at least 3, or at least 5, or at least 7, or 10 or more amino acids located on the keratin 19 moiety of the heterotypic complex of keratin 7 with keratin 19.

11. The method according to any one of claims 7-10, wherein the first antibody specifically binds amino acid sequence 300-350 on keratin 7, and the second antibody specifically binds amino acid sequence 346-367 on keratin 19.

12. The method according to any one of claims 7-11, wherein the first antibody is the Ks 7.18 monoclonal antibody and the second antibody is the BM-19.21 monoclonal antibody.

13. The method according to any one of claims 7-10, wherein the first antibody is the RCK105 monoclonal antibody and the second antibody is the Ks 19.1 monoclonal antibody.

14. The method according to any one of claims 7-13, further comprising a step of comparing an expression of heterotypic complex of keratin 7 with keratin 19 and/or fragment(s) thereof to a positive and/or negative control.

15. The method according to any one of claims 7-14, further comprising a step of scoring the amount of heterotypic complex of keratin 7 with keratin 19 and/or fragment(s) thereof.

16. A composition according to claims 1-6, for use in the *in vitro* method of claims 7-15 for
i) diagnosing and/or prognosing malignant neoplastic disease in a subject, and/or
ii) predicting efficacy of treatment of malignant neoplastic disease in a subject, and/or
iii) assessing outcome of treatment of malignant neoplastic disease in a subject, and/or
iv) assessing recurrence of malignant neoplastic disease in a subject
wherein the subject is a mammal having, or is suspected of having, a malignant neoplastic disease.

17. The composition for use according to claim 16, wherein the malignant neoplastic disease is selected from the group consisting of: bile duct cancer (extrahepatic), bladder cancer, breast cancer, carcinoma of unknown (primary), cervical cancer, colon cancer, endometrial cancer, esophageal cancer, gallbladder cancer, gastric (stomach) cancer, head and neck cancer, hepatocellular (liver) cancer, hypopharyngeal cancer, kidney cancer, laryngeal cancer, liver cancer (primary), lung cancer (non-small cell), lung cancer (small cell), mesothelioma, non-small cell lung cancer, ovarian cancer, ovarian epithelial cancer (surface epithelial-stromal tumor), ovarian germ cell tumor, ovarian low malignant potential tumor, pancreatic cancer, prostate cancer, rectal cancer, renal cell carcinoma (kidney cancer), salivary gland cancer, small cell lung cancer, small intestine cancer, stomach cancer, testicular cancer, thyroid cancer, transitional cell cancer of the renal pelvis and ureter, uterine cancer (endometrial), preferably the malignant neoplastic disease is selected from lung cancer, bladder cancer, esophagus cancer, hepatocellular cancer, pancreatic cancer, testicular cancer and ovarian cancer, more preferably the malignant neoplastic disease is selected from lung cancer, bladder cancer, esophagus cancer, and ovarian cancer.

18. A kit for
i) detecting heterotypic complex of keratin 7 with keratin 19 and/or fragment(s) thereof in a biological sample; or
ii) detecting malignant neoplastic disease in a subject; or
iii) diagnosing or prognosing malignant neoplastic disease in a subject; or
iv) predicting outcome of treatment of malignant neoplastic disease in a subject; or
v) assessing efficacy of treatment of malignant neoplastic disease in a subject; or
vi) assessing recurrence of malignant neoplastic disease in a subject;
the kit comprising:
a) a composition as defined in any one of claims 1-6; or alternatively
b) a first container comprising a first antibody, antigen-binding fragment, or combination thereof recognizing a keratin 7 peptide and/or fragment(s) thereof; and
c) a second container comprising a second antibody, antigen-binding fragment, or combination thereof recognizing a keratin 19 peptide and/or fragment thereof, and
d) optionally instructions for performing a method as defined in any one of claims 7-15.

## Patentansprüche

1. Zusammensetzung, umfassend zumindest zwei Antikörper, Antigenbindungsfragment(e) oder Kombinationen davon, wobei zumindest ein erster Antikörper, ein Antigenbindungsfragment oder eine Kombination davon eine Sequenz erkennt, die innerhalb der 256-412 Aminosäuresequenz, oder bevorzugter innerhalb der 300-380 Aminosäuresequenz in der funktionellen Keratin-7-Gruppe des heterotypischen Komplexes von Keratin 7 mit Keratin 19 und/oder Fragment(en) davon gelegen ist, und zumindest ein zweiter Antikörper, ein Antigenbindungsfragment oder eine Kombination davon eine Sequenz erkennt, die innerhalb der 244-400 Aminosäuresequenz, oder bevorzugter innerhalb der 311-375 Aminosäuresequenz in der funktionellen Keratin-19-Gruppe des heterotypischen Komplexes von Keratin 7 mit Keratin 19 und/oder Fragment(en) davon gelegen ist, **dadurch gekennzeichnet, dass** die ersten und zweiten Antikörper, Antigenbindungsfragment(e) oder Kombination davon fähig sind, sich spezifisch und gleichzeitig an den heterotypischen Komplex von Keratin 7 mit Keratin 19 und/oder Fragment(en) davon zu binden.

2. Zusammensetzung nach Anspruch 1, wobei der erste Antikörper aus der Gruppe ausgewählt ist, bestehend aus den Antikörpern KS 7.18 und RCK105 oder Antigenbindungsfragment(en)-varianten, -fusionen, -derivaten oder Kombinationen davon, und eine Sequenz von zumindest 3 oder zumindest 5 oder zumindest 7 oder 10 oder mehr Aminosäuren erkennt, die auf der funktionellen Keratin-7-Gruppe des heterotypischen Komplexes von Keratin 7 mit Keratin 19 gelegen ist.

3. Zusammensetzung nach einem der Ansprüche 1-2, wobei der zweite Antikörper aus der Gruppe ausgewählt ist, bestehend aus den Antikörpern A53-B/A2aka Ks19.1, BM-19.21, KS19.2 oder Antigenbindungsfragment(en)-varianten, -fusionen, -derivaten oder einer Kombination davon, und eine Sequenz von zumindest 3 oder zumindest 5 oder zumindest 7 oder 10 oder mehr Aminosäuren erkennt, die auf der funktionellen Keratin-19-Gruppe des heterotypischen Komplexes von Keratin 7 mit Keratin 19 gelegen ist.

4. Zusammensetzung nach einem der Ansprüche 1-3, wobei der erste Antikörper Aminosäuresequenz 300-350 auf Keratin 7 spezifisch bindet und der zweite Antikörper Aminosäure 346-367 auf Keratin 19 spezifisch bindet.

5. Zusammensetzung nach einem der Ansprüche 1-4, wobei der erste Antikörper der Ks 7.18 monoklonale Antikörper ist und der zweite Antikörper der BM-19.21 monoklonale Antikörper ist.

6. Zusammensetzung nach einem der Ansprüche 1-3, wobei der erste Antikörper der RCK105 monoklonale Antikörper ist und der zweite Antikörper der Ks 19.1 monoklonale Antikörper ist.

7. *In-vitro*-Verfahren zum Detektieren eines heterotypischen Komplexes von Keratin 7 mit Keratin 19 und/oder Fragment(en) davon in einer biologischen Probe, das Verfahren die Schritte umfassend von:
a) Kontaktieren der biologischen Probe mit einer Zusammensetzung, umfassend zumindest zwei Antikörper, Antigenbindungsfragment(e) oder Kombinationen davon, wobei zumindest ein erster Antikörper, ein Antigenbindungsfragment oder eine Kombination davon eine Sequenz erkennt, die innerhalb der 256-412 Aminosäuresequenz, oder bevorzugter innerhalb der 300-380 Aminosäuresequenz in der funktionellen Keratin-7-Gruppe des heterotypischen Komplexes von Keratin 7 mit Keratin 19 und/oder Fragment(en) davon gelegen ist, und zumindest ein zweiter Antikörper, ein Antigenbindungsfragment oder eine Kombination davon eine Sequenz erkennt, die innerhalb der 244-400 Aminosäuresequenz, oder bevorzugter innerhalb der 311-375 Aminosäuresequenz in der funktionellen Keratin-19-Gruppe des heterotypischen Komplexes von Keratin 7 mit Keratin 19 und/oder Fragment(en) davon gelegen ist, wobei die ersten und zweiten Antikörper, Antigenbindungsfragment(e) oder Kombination davon fähig sind, spezifisch und zeitgleich an den heterotypischen Komplex von Keratin 7 mit Keratin 19 und/oder Fragment(en) davon zu binden; oder alternativ
b) Kontaktieren der biologischen Probe mit einem ersten Antikörper, einem Antigenbindungsfragment oder einer Kombination davon, wobei eine Sequenz erkannt wird, die innerhalb der 256-412 Aminosäuresequenz, oder bevorzugter innerhalb der 300-380 Aminosäuresequenz in der funktionellen Keratin-7-Gruppe des heterotypischen Komplexes von Keratin 7 mit Keratin 19 und/oder Fragment(en) davon gelegen ist; und
c) Kontaktieren der biologischen Gruppe mit einem zweiten Antikörper, einem Antigenbindungsfragment oder einer Kombination davon, wobei eine Sequenz erkannt wird, die innerhalb der 244-400 Aminosäuresequenz, oder bevorzugter innerhalb der 311-375 Aminosäuresequenz in der funktionellen Keratin-19-Gruppe des heterotypischen Komplexes von Keratin 7 mit Keratin 19 und/oder Fragment(en) davon gelegen ist; und
d) Detektieren des heterotypischen Komplexes von Keratin 7 mit Keratin 19 und/oder Fragment(en) davon;
wobei Schritte b) und c) in jeder Reihenfolge oder zeitgleich ausgeführt werden können.

8. Verfahren nach Anspruch 7, wobei die biologische Probe eine biologische Fluidprobe der Gruppe, bestehend aus Blutserum, Blutplasma, Lymphflüssigkeit, Exsudaten, Exkrementen, Magensäure, Magensaft, Lymphflüssigkeit, Schleim, Perikardflüssigkeit, Peritonealflüssigkeit, Pleuralflüssigkeit, Eiter, Speichel, Auswurf, Gelenksflüssigkeit, Tränen, Schweiß, Scheidensekret, Erbrochenem und Urin ist, wobei die biologische Fluidprobe vorzugsweise aus Blut, Serum und/oder Plasma ausgewählt ist.

9. Verfahren nach einem der Ansprüche 7-8, wobei der erste Antikörper aus der Gruppe ausgewählt ist, bestehend aus den Antikörpern KS 7.18 und RCK105 oder Antigenbindungsfragment(en)-varianten, -fusionen, -derivaten oder einer Kombination davon, und eine Sequenz von zumindest 3 oder zumindest 5 oder zumindest 7 oder 10 oder mehr Aminosäuren erkennt, die auf der funktionellen Keratin-7-Gruppe des heterotypischen Komplexes von Keratin 7 mit Keratin 19 gelegen ist.

10. Verfahren nach einem der Ansprüche 7-8, wobei der zweite Antikörper aus der Gruppe ausgewählt ist, bestehend aus den Antikörpern A53-B/A2aka Ks19.1, BM-19.21, Ks19.2 oder Antikörperfragment(en)-varianten, -fusionen, -derivaten oder einer Kombination davon, und eine Sequenz von zumindest 3 oder zumindest 5 oder zumindest 7, oder 10 oder mehr Aminosäuren erkennt, die auf der funktionellen Keratin-19-Gruppe des heterotypischen Komplexes von Keratin 7 mit Keratin 19 gelegen ist.

11. Verfahren nach einem der Ansprüche 7-10, wobei der erste Antikörper Aminosäuresequenz 300-350 auf Keratin 7 spezifisch bindet und der zweite Antikörper Aminosäuresequenz 346-367 auf Keratin 19 spezifisch bindet.

12. Verfahren nach einem der Ansprüche 7-11, wobei der erste Antikörper der Ks 7.18 monoklonale Antikörper ist und der zweite Antikörper der BM-19.21 monoklonale Antikörper ist.

13. Verfahren nach einem der Ansprüche 7-10, wobei der erste Antikörper der RCK105 monoklonale Antikörper ist und der zweite Antikörper der Ks 19.1 monoklonale Antikörper ist.

14. Verfahren nach einem der Ansprüche 7-13, weiter umfassend einen Schritt zum Vergleichen eines Ausdrucks eines heterotypischen Komplexes von Keratin 7 mit Keratin 19 und/oder Fragment(en) davon mit einer positiven und/oder negativen Kontrolle.

15. Verfahren nach einem der Ansprüche 7-14, weiter umfassend einen Schritt zum Beurteilen der Menge von heterotypischem Komplex von Keratin 7 mit Keratin 19 und/oder Fragment(en) davon.

16. Zusammensetzung nach Ansprüche 1-6, zur Verwendung im *in-vitro*-Verfahren nach Ansprüche 7-15 zum
i. Diagnostizieren und/oder Prognostizieren einer bösartigen neoplastischen Erkrankung in einem Subjekt und/oder
ii. Vorhersagen einer Wirksamkeit einer Behandlung einer bösartigen neoplastischen Erkrankung in einem Subjekt und/oder
iii. Bewerten eines Behandlungsergebnisses einer bösartigen neoplastischen Erkrankung in einem Subjekt und/oder
iv. Bewerten eines Wiederauftretens einer bösartigen neoplastischen Erkrankung in einem Subjekt, wobei das Subjekt ein Säugetier mit einer bösartigen neoplastischen Erkrankung ist oder unter Verdacht steht, an dieser zu leiden.

17. Zusammensetzung zur Verwendung nach Anspruch 16, wobei die bösartige neoplastische Erkrankung aus der Gruppe ausgewählt ist, bestehend aus: Gallengangkrebs (extrahepatisch), Blasenkrebs, Brustkrebs, unbekanntem Karzinom (primär), Gebärmutterhalskrebs, Darmkrebs, Endometriumkrebs, Speiseröhrenkrebs, Gallenblasenkrebs, Magenkrebs, Kopf- und Halskrebs, hepatozellulärem (Leber-) Krebs, Hypopharynxkrebs, Nierenkrebs, Kehlkopfkrebs, Leberkrebs (primär), Lungenkrebs (nicht kleinzellig), Lungenkrebs (kleinzellig), Mesotheliom, nicht kleinzelligem Lungenkrebs, Eierstockkrebs, Eierstockepithelkrebs (Oberflächenepithalstromatumor), Eierstockkeimzellentumor, Eierstocktumor mit niedrigem bösartigen Potential, Bauchspeicheldrüsenkrebs, Prostatakrebs, Rektalkrebs, Nierenzellkarzinom (Nierenkrebs), Speicheldrüsenkrebs, kleinzelligem Lungenkrebs, Dünndarmkrebs, Magenkrebs, Hodenkrebs, Schilddrüsenkrebs, Übergangs-Zellkarzinom des Nierenbeckens und Harnleiters, Gebärmutterkrebs (endometrial), wobei vorzugsweise die bösartige neoplastische Erkrankung ausgewählt ist aus Lungenkrebs, Blasenkrebs, Speiseröhrenkrebs, hepatozellulärem Krebs, Bauchspeicheldrüsenkrebs, Hodenkrebs und Eierstockkrebs, bevorzugter die bösartige neoplastische Erkrankung ausgewählt ist aus Lungenkrebs, Blasenkrebs, Speiseröhrenkrebs und Eierstockkrebs.

18. Kit zum
i. Detektieren eines heterotypischen Komplexes von Keratin 7 mit Keratin 19 und/oder Fragment(en) davon in einer biologischen Probe; oder
ii. Detektieren einer bösartigen neoplastischen Erkrankung in einem Subjekt; oder
iii. Diagnostizieren oder Prognostizieren einer bösartigen neoplastischen Erkrankung in einem Subjekt; oder
iv. Vorhersagen eines Behandlungsergebnisses einer bösartigen neoplastischen Erkrankung in einem Subjekt; oder
v. Bewerten einer Wirksamkeit einer Behandlung einer bösartigen neoplastischen Erkrankung in einem Subjekt; oder
vi. Bewerten eines Wiederauftretens einer bösartigen neoplastischen Erkrankung in einem Subjekt;
der Kit umfassend:
a) eine Zusammensatzung nach einem der Ansprüche 1-6; oder alternativ
b) einen ersten Behälter, umfassend einen ersten Antikörper, ein Antigenbindungsfragment oder eine Kombination davon, die ein Keratin-7-peptid und/oder Fragment(e) davon erkennen; und
c) einen zweiten Behälter, umfassend einen zweiten Antikörper, Antigenbindungsfragment oder eine Kombination davon, die ein Keratin-19-petid und/oder Fragment davon erkennen, und
d) optional Anweisungen zum Ausführen eines Verfahrens nach einem der Ansprüche 7-15.

## Revendications

1. Composition comprenant au moins deux anticorps, un ou des fragments de liaison à l'antigène ou leurs combinaisons, dans laquelle au moins un premier anticorps, un fragment de liaison à l'antigène ou leurs combinaisons reconnaît une séquence localisée dans la séquence de 256-412 acides aminés ou, mieux encore, dans la séquence de 300-380 acides aminés dans la fraction de kératine 7 du complexe hétérotypique de kératine 7 avec la kératine 19 et/ou un ou des fragments de celui-ci, et au moins un second anticorps, un fragment de liaison à l'antigène ou leurs combinaisons reconnaît une séquence localisée dans la séquence de 244-400 acides aminés ou, mieux encore, dans la séquence de 311-375 acides aminés dans le groupe de kératine 19 du complexe hétérotypique de kératine 7 avec la kératine 19 et/ou un ou des fragments de celui-ci, **caractérisée en ce que** lesdits premier et second anticorps, le ou les fragments de liaison à l'antigène ou leurs combinaisons sont capables de se lier spécifiquement et de manière simultanée à un complexe hétérotypique de kératine 7 avec la kératine 19 et/ou un ou des fragments de celui-ci.

2. Composition selon la revendication 1, dans laquelle le premier anticorps est choisi dans le groupe constitué des anticorps KS 7.18 et RCK105 ou des variants, fusions, dérivés d'un ou plus de fragments de liaison à l'antigène ou leurs combinaisons et reconnaît une séquence d'au moins 3, ou d'au moins 5 ou d'au moins 7 ou de 10 ou plus d'acides aminés localisée dans la fraction de kératine 7 du complexe hétérotypique de kératine 7 avec la kératine 19.

3. Composition selon l'une quelconque des revendications 1 et 2, dans laquelle le second anticorps est choisi dans le groupe constitué des anticorps A53-B/A2aka Ks19.1, BM-19-21, Ks19.2 ou de variants, de fusions, de dérivés de fragment(s) de liaison à l'antigène ou leurs combinaisons et reconnaît une séquence d'au moins 3 ou d'au moins 5 ou d'au moins 7 ou de 10 ou plus d'acides aminés localisée dans la fraction de kératine 19 du complexe hétérotypique de la kératine 7 avec la kératine 19.

4. Composition selon l'une quelconque des revendications 1 à 3, dans laquelle le premier anticorps se lie spécifiquement à une séquence de 300-350 acides aminés sur la kératine 7 et le second anticorps se lie spécifiquement à une séquence de 346-367 acides aminés sur la kératine 19.

5. Composition selon l'une quelconque des revendications 1 à 4, dans laquelle le premier anticorps est l'anticorps monoclonal KS 7.18 et le second anticorps est l'anticorps monoclonal BM-19.21.

6. Composition selon l'une quelconque des revendications 1 à 3, dans laquelle le premier anticorps est l'anticorps monoclonal RCK105 et le second anticorps est l'anticorps monoclonal KS 19.1.

7. Procédé *in vivo* de détection d'un complexe hétérotypique de kératine 7 avec la kératine 19 et/ou de leur(s) fragment(s) dans un échantillon biologique, ledit procédé comprenant les étapes consistant à :
a) mettre en contact ledit échantillon biologique avec une composition comprenant au moins deux anticorps, un ou des fragments de liaison à l'antigène ou leurs combinaisons, dans lequel au moins un premier anticorps, un fragment de liaison à l'antigène ou leurs combinaisons reconnaît une séquence localisée dans la séquence de 256-412 acides aminés ou, mieux encore, dans la séquence de 300-380 acides aminés de la fraction de kératine 7 du complexe hétérotypique de kératine 7 avec la kératine 19 et/ou de leur(s) fragment(s), et au moins un second anticorps, un fragment de liaison à l'antigène ou leurs combinaisons reconnaît une séquence localisée dans la séquence de 244-400 acides aminés ou, mieux encore, dans la séquence de 311-375 acides aminés de la fraction de kératine 19 du complexe hétérotypique de kératine 7 avec la kératine 19 et/ou de leur(s) fragment(s), lesdits premier et second anticorps, le ou les fragments de liaison à l'antigène ou leurs combinaisons sont capables de se lier spécifiquement et de manière simultanée à un complexe hétérotypique de kératine 7 avec la kératine 19 et/ou à leur(s) fragment(s) ; ou, en variante,
b) mettre en contact ledit échantillon biologique avec un premier anticorps, un fragment de liaison à l'antigène ou leurs combinaisons reconnaît une séquence localisée dans la séquence de 256-412 acides aminés ou, mieux encore, dans la séquence de 300-380 acides aminés de la fraction de kératine 7 du complexe hétérotypique de kératine 7 avec la kératine 19 et/ou leur(s) fragment(s) ; et
c) mettre en contact ledit échantillon biologique avec un second anticorps, un fragment de liaison à l'antigène ou leurs combinaisons reconnaît une séquence localisée dans la séquence de 244-400 acides aminés ou, mieux encore, dans la séquence de 311-375 acides aminés de la fraction de kératine 19 du complexe hétérotypique de kératine 7 avec la kératine 19 et/ou leur(s) fragment(s) ; et
d) détecter ledit complexe hétérotypique de kératine 7 avec la kératine 19 et/ou leur(s)s fragment(s) ;
dans lequel les étapes b) et c) peuvent être effectuées dans un ordre quelconque ou simultanément.

8. Procédé selon la revendication 7, dans lequel l'échantillon biologique est un échantillon de fluide biologique du groupe constitué du sérum sanguin, du plasma sanguin, de la lymphe, d'exsudats, de fèces, d'acide gastrique, de suc gastrique, de lymphe, de mucus, de fluide péricardique, de fluide péritonéal, de fluide pleural, de pus, de salive, de crachat, de fluide synovial, de larmes, de sueur, de sécrétion vaginale, de vomissure et d'urine, de préférence l'échantillon de fluide biologique est choisi parmi le sang, le sérum et/ou le plasma.

9. Procédé selon l'une quelconque des revendications 7 et 8, dans lequel le premier anticorps est choisi dans le groupe constitué des anticorps KS 7.18 et RCK105 ou de variants, de fusions, de dérivés de leur(s) fragment(s) de liaison à l'antigène ou de leurs combinaisons et reconnaît une séquence d'au moins 3 ou au d'au moins 5 ou d'au moins 7 ou de 10 ou plus d'acides aminés localisée dans le groupe de kératine 7 du complexe hétérotypique de kératine 7 avec la kératine 19.

10. Procédé selon l'une quelconque des revendications 7 et 8, dans lequel le second anticorps est choisi dans le groupe constitué des anticorps A53-B/2aka Ks19.1, BM-19.21, Ks19.2 ou de variants, de fusions, de dérivés de fragments d'anticorps ou leur combinaisons et reconnaît une séquence d'au moins 3 ou d'au moins 5 ou d'au moins 7 ou de 10 ou plus d'acides aminés localisée dans le groupe de kératine 19 du complexe hétérotypique de la kératine 7 avec la kératine 19.

11. Procédé selon l'une quelconque des revendications 7 à 10, dans lequel le premier anticorps se lie spécifiquement à une séquence de 300-350 acides aminés sur la kératine 7 et le second anticorps se lie spécifiquement à une séquence de 346-367 acides aminés sur la kératine 19.

12. Procédé selon l'une quelconque des revendications 7 à 11, dans lequel le premier anticorps est l'anticorps monoclonal Ks 7.18 et le second anticorps est l'anticorps monoclonal BM-19.21.

13. Procédé selon l'une quelconque des revendications 7 à 10, dans lequel le premier anticorps est l'anticorps monoclonal RCK105 et le second anticorps est l'anticorps monoclonal Ks 19.1.

14. Procédé selon l'une quelconque des revendications 7 à 13, comprenant en outre une étape de comparaison d'une expression du complexe hétérotypique de kératine 7 avec la kératine 19 et/ou d'un ou plus de leurs fragments à un témoin positif et/ou un témoin négatif.

15. Procédé selon l'une quelconque des revendications 7 à 14, comprenant en outre une étape de comptage de la quantité de complexe hétérotypique de kératine 7 avec la kératine 19 et/ou d'un ou plus de leurs fragments.

16. Composition selon les revendications 1 à 6 pour utilisation dans le procédé *in vitro* des revendications 7 à 15 pour
i) le diagnostic et/ou le pronostic d'une maladie néoplasique maligne chez un sujet et/ou
ii) la prédiction de l'efficacité du traitement de la maladie néoplasique maligne chez un sujet et/ou
iii) l'évaluation du résultat de traitement de la maladie néoplasique maligne chez un sujet et/ou
iv) l'évaluation de la récurrence de la maladie néoplasique maligne chez un sujet,
dans lequel le sujet est un mammifère ayant ou suspecté d'avoir une maladie néoplasique maligne.

17. Composition pour utilisation selon la revendication 16, dans lequel la maladie néoplasique maligne est choisie dans le groupe constitué des suivantes : cancer des canaux biliaires (extrahépatique), cancer de la vessie, cancer du sein, carcinome de cause inconnue (primaire), cancer cervical, cancer du côlon, cancer endométrial, cancer de l'oesophage, cancer de la vésicule biliaire, cancer gastrique (estomac), cancer de la tête et du cou, cancer hépatocellulaire (foie), cancer hypopharyngien, cancer des reins, cancer laryngien, cancer du foie (primaire), cancer des poumons (cellules non petites), cancer des poumons (cellules petites), mésothéliome, cancer des poumons à cellules non petites, cancer ovarien, cancer épithélial ovarien (tumeur épithéliale-stromale de surface), tumeurs embryonnaires et germinales ovariennes, tumeur potentielle maligne ovarienne faible, cancer du pancréas, cancer de la prostate, cancer rectal, carcinome de cellules rénales (cancer des reins), cancer de la glande salivaire, cancer des poumons à petites cellules, cancer du petit intestin, cancer de l'estomac, cancer des testicules, cancer de la thyroïde, cancer des cellules de transition du pelvis rénal et de l'urètre, cancer utérin (endométrial), de préférence la maladie néoplasique maligne est choisie parmi le cancer des poumons, le cancer de la vessie, le cancer de l'oesophage, le cancer hépatocellulaire, le cancer du pancréas, le cancer des testicules et le cancer ovarien, mieux encore, la maladie néoplasique maligne est choisie parmi le cancer des poumons, le cancer de la vessie, le cancer de l'oesophage et le cancer ovarien.

18. Kit pour
i) détecter un complexe hétérotypique de la kératine 7 avec la kératine 19 et/ou un ou des fragments de celui-ci dans un échantillon biologique ; ou
ii) détecter une maladie néoplasique maligne chez un sujet ; ou
iii) diagnostiquer ou pronostiquer une maladie néoplasique maligne chez un sujet ; ou
iv) prédire le résultat du traitement d'une maladie néoplasique maligne chez un sujet ; ou
v) évaluer l'efficacité du traitement d'une maladie néoplasique maligne chez un sujet ; ou
vi) évaluer la récurrence d'une maladie néoplasique maligne chez un sujet ;
le kit comprenant :
a) une composition telle que définie selon l'une quelconque des revendications 1 à 6 ; ou en variante
b) un premier réceptacle comprenant un premier anticorps, un fragment de liaison à l'antigène ou une de leurs combinaisons reconnaissant un peptide de kératine 7 et/ou un ou des fragments de celui-ci ; et
c) un second réceptacle comprenant un second anticorps, un fragment de liaison à l'antigène ou une de leurs combinaisons reconnaissant un peptide de kératine 19 et/ou un fragment de celui-ci et
d) éventuellement des instructions pour effectuer un procédé tel que défini dans l'une quelconque des revendications 7 à 15.
